(19) Europäisches Patentamt
European Patent Office
Office européen des brevets

(11) **EP 2 700 633 A1**

(12) **EUROPÄISCHE PATENTANMELDUNG**

(43) Veröffentlichungstag:
**26.02.2014 Patentblatt 2014/09**

(21) Anmeldenummer: **12181442.0**

(22) Anmeldetag: **23.08.2012**

(51) Int Cl.:
***C07D 215/38*** (2006.01) ***C07D 317/36*** (2006.01)
***C07D 317/46*** (2006.01) ***C07D 471/04*** (2006.01)
***C08G 64/02*** (2006.01) ***C08G 64/34*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Bayer MaterialScience AG**
**51373 Leverkusen (DE)**

(72) Erfinder:
- **Müller, Thomas, Ernst, Dr.**
  **52062 Aachen (DE)**
- **Gürtler, Christoph, Dr.**
  **50676 Köln (DE)**
- **Elmas, Sait, Dr.**
  **52477 Alsdorf (DE)**
- **Köhler, Burkhard, Dr.**
  **34289 Zierenberg (DE)**
- **Leitner, Walter, Prof. Dr.**
  **52074 Aachen (DE)**
- **Harrer, Markus, Dr.**
  **35039 Marburg (DE)**
- **Sundermeyer, Jörg, Prof. Dr.**
  **35041 Marburg (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(54) **Verfahren zur Herstellung von linearen und/oder cyclischen Carbonatestern**

(57) Die vorliegende Erfindung betrifft ein Verfahren zur Herstellung von linearen und/oder cyclischen Carbonatestern, umfassend den Schritt der Reaktion eines Epoxids mit Kohlendioxid in Gegenwart eines Katalysators. Sie betrifft weiterhin die Verwendung spezieller Katalysatoren für die Reaktion von Epoxiden mit Kohlendioxid, spezielle Katalysatoren sowie spezielle Reaktionsprodukte. Der Katalysator umfasst einen Komplex eines Metalls M oder M-A mit einem Liganden L, wobei das Metall M in einer Oxidationsstufe von ≥ 0 vorliegt, A für Halogenid, Carboxylat, Phenolat, Sulfonat, Phosphonat, Alkyl, Alkoxy oder Amido steht und der Ligand L die nachfolgende Struktur (Ia) oder (Ib) aufweist, wobei weiterhin eine oder beide der in (Ia) und/oder (Ib) gezeigten OH-Gruppen deprotoniert sein können:

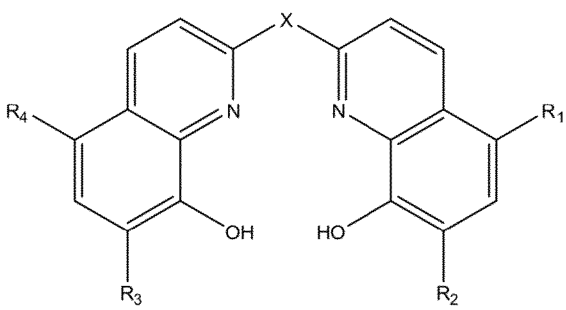

(Ia)

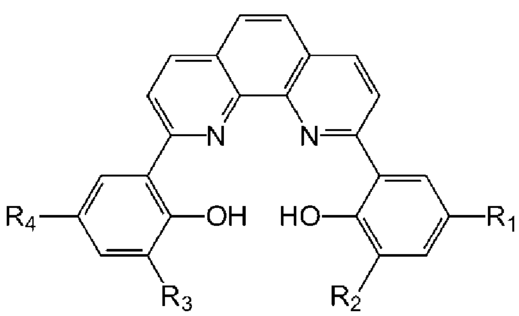

(Ib)

EP 2 700 633 A1

**Beschreibung**

Die vorliegende Erfindung betrifft ein Verfahren zur Herstellung von linearen und/oder cyclischen Carbonatestern, umfassend den Schritt der Reaktion eines Epoxids mit Kohlendioxid in Gegenwart eines Katalysators. Sie betrifft weiterhin die Verwendung spezieller Katalysatoren für die Reaktion von Epoxiden mit Kohlendioxid, spezielle Katalysatoren sowie spezielle Reaktionsprodukte.

Die Reaktion von Epoxiden mit Kohlendioxid ist unter mehreren Aspekten interessant. Zum einen dient diese Reaktion ganz allgemein der Fixierung von $CO_2$, welches beispielsweise bei der Reinigung von Abgasen aus Verbrennungsanlagen anfällt. Weiterhin lassen sich lineare und/oder cyclische Carbonate (Carbonatester) erhalten. Lineare Carbonatester finden Anwendung als polymere Werkstoffe. Cyclische Carbonatester können unter anderem als Lösungsmittel, als Elektrolyt in Batterien oder als Transesterifizierungsreagenz verwendet werden. Chemical Communications 47(2011)141-163 gibt eine Übersicht über die bekannten Katalysatoren für die Copolymerisation von Epoxiden und $CO_2$.

In Bezug auf eine weitere Katalysatorklasse offenbart WO 2009/095164 A1 ein Verfahren zur Herstellung von Urethanen durch oxidative Carbonylierung von Aminoverbindungen in Gegenwart von Kohlenmonoxid, Sauerstoff und organischen, Hydroxylgruppen tragenden Verbindungen. Die Carbonylierung wird in Abwesenheit von halogenhaltigen Promotoren und in Anwesenheit von Metallkomplexkatalysatoren durchgeführt, die neutrale zweizähnige N-Chelatliganden des Typs (N~N), zwei monoanionische N,O-Chelatliganden des allgemeinen Typs $(N\sim O)^-$ oder vierzähnige dianionische Chelatliganden $(O\sim N\sim N\sim)^{2-}$ enthalten.

WO 2008/135337 A1 beschreibt Bis(hydroxychinolin)-Metallkomplexe sowie deren Verwendung als Bleichkatalysatoren.

Mit den derzeit eingesetzten Katalysatorsystemen für die Copolymerisation von Epoxiden mit Kohlendioxid wird eine technisch anwendbare Aktivität erst bei erhöhten Temperaturen erreicht. Auch weisen die resultierenden Polycarbonate in der Regel eine breite Verteilung der Molmassen (hohe Polydispersität, PDI) auf. Weiterhin ist die technische Zugänglichkeit vieler beschriebener Katalysatorkomplexe nicht optimal.

Die vorliegende Erfindung hat sich die Aufgabe gestellt, die angesprochenen Nachteile im Stand der Technik zumindest teilweise zu beheben. Insbesondere hat sie sich die Aufgabe gestellt, ein Verfahren zur Reaktion von Epoxiden mit Kohlendioxid bereitzustellen, bei dem der Energieeinsatz geringer ist und der Katalysator leicht erhalten werden kann.

Erfindungsgemäß wurde diese Aufgabe gelöst durch ein Verfahren zur Herstellung von linearen und/oder cyclischen Carbonatestern, umfassend den Schritt der Reaktion eines Epoxids mit Kohlendioxid in Gegenwart eines Katalysators, wobei der Katalysator einen Komplex eines Metalls M oder M-A mit einem Liganden L umfasst, wobei das Metall M in einer Oxidationsstufe von $\geq 0$ vorliegt,

A für Halogenid, Carboxylat, Phenolat, Sulfonat, Phosphonat, Alkyl, Alkoxy oder Amido steht und der Ligand L die nachfolgende Struktur (Ia) oder (Ib) aufweist, wobei weiterhin eine oder beide der in (Ia) und/oder (Ib) gezeigten OH-Gruppen deprotoniert sein können:

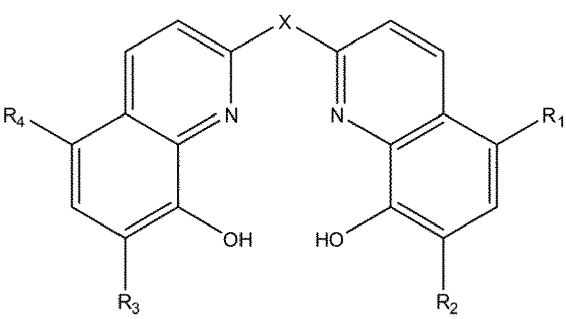

(Ia)

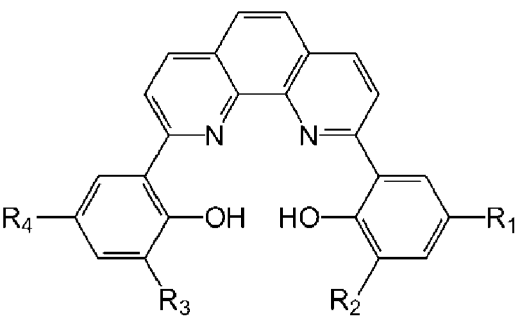

(Ib)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen und X für eine Brücke der Formel (II) oder (III) steht:

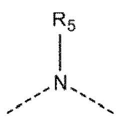

(II)

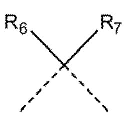

(III)

wobei R5 für einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest, oder einen gegebenenfalls Alkyl-substituierten Pyridylrest oder Pyridylmethylrest steht und R6 und R7 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest oder einen -COOR8-Rest stehen, wobei R8 für einen $C_1$-$C_8$-Alkylrest steht.

Der Ausdruck "Alkyl" umfasst im Sinne dieser Erfindung acyclische gesättigte oder ungesättigte aliphatische Kohlenwasserstoffreste, die jeweils verzweigt oder unverzweigt sowie unsubstituiert oder ein- oder mehrfach substituiert sein können. Bevorzugt ist "Alkyl" aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, n-Heptyl, n-Octyl, n-Nonyl, n-Decyl, Ethenyl (Vinyl), Ethinyl, Propenyl ($-CH_2CH=CH_2$, $-CH=CH-CH_3$, $-C(=CH_2)-CH_3$), Propinyl ($-CH_2-C\equiv CH$, $-C\equiv C-CH_3$), Butenyl, Butinyl, Pentenyl, Pentinyl, Hexenyl und Hexinyl, Heptenyl, Heptinyl, Octenyl, Octinyl, Nonenyl, Noninyl, Decenyl und Decinyl umfasst.

Der Ausdruck "Cycloalkyl" bedeutet für die Zwecke dieser Erfindung cyclische aliphatische (cycloaliphatische) Kohlenwasserstoffe, wobei die Kohlenwasserstoffe gesättigt oder ungesättigt (aber nicht aromatisch), unsubstituiert oder ein- oder mehrfach substituiert sein können. Die Bindung des Cycloalkyls an die jeweilige übergeordnete allgemeine Struktur kann über jedes beliebige und mögliche Ringglied des Cycloalkyl-Restes erfolgen. Die Cycloalkyl-Reste können auch mit weiteren gesättigten, (partiell) ungesättigten, (hetero)cyclischen, aromatischen oder heteroaromatischen Ringsystemen, d.h. mit Cycloalkyl, Heterocyclyl, Aryl oder Heteroaryl kondensiert sein, die wiederum unsubstituiert oder einfach oder mehrfach substituiert sein können. Die Cycloalkyl-Reste können weiterhin einfach oder mehrfach verbrückt sein wie beispielsweise. im Fall von Adamantyl, Bicyclo[2.2.1 ]heptyl oder Bicyclo[2.2.2]octyl. Bevorzugt ist Cycloalkyl aus der Gruppe ausgewählt, die Cyclopropyl, Cyclobutyl, Cyclopentyl, Cyclohexyl, Cycloheptyl, Cyclooctyl, Cyclononyl, Cyclodecyl, Adamantyl,

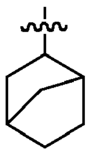 , 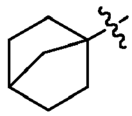 , 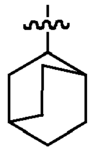 ,

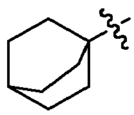 ,

Cyclopentenyl, Cyclohexenyl, Cycloheptenyl und Cyclooctenyl umfasst.

Der Begriff "Aryl" bedeutet im Sinne dieser Erfindung aromatische Kohlenwasserstoffe, unter anderem Phenyle und Naphthyle. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Die Bindung des Aryls an die übergeordnete allgemeine Struktur kann über jedes beliebige und mögliche Ringglied des Aryl-Restes erfolgen. Die Aryl-Reste können auch mit weiteren gesättigten, (partiell) ungesättigten, (hetero)cyclischen, aromatischen oder heteroaromatischen Ringsystemen kondensiert sein, d.h. mit Cycloalkyl, Heterocyclyl, Aryl oder Heteroaryl, die wiederum unsubstituiert oder einfach oder mehrfach substituiert sein können. Beispiele für kondensierte Aryl-Reste sind Benzodioxolanyl und Benzodioxanyl. Bevorzugt ist Aryl aus der Gruppe ausgewählt, die Phenyl, 1-Naphthyl und 2-Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

In entsprechender Weise leiten sich die Begriffe "Aralkyl" und "Alkylaryl" aus Kombinationen von "Alkyl" und "Aryl" ab.

Allgemein können für das erfindungsgemäße Verfahren Epoxide mit 2-24 Kohlenstoffatomen eingesetzt werden. Bei Epoxiden mit 2-24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Limonenoxid, Styroloxid, Methylstyroloxid, Pinenoxid, ein- oder mehrfach epoxidierte Fette als Mono-, Di- und Triglyceride, epoxidierte Fettsäuren,

C1-C24-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkoxysilane wie beispielsweise 3-Glycidyloxypropyltrimethoxysilan, 3-Glycidyloxypropyltriethoxysilan, 3-Glycidyloxypropyltripropoxysilan, 3-Glycidyloxypropyl-methyl-dimethoxysilan, 3-Glycidyl-oxypropyl-ethyldiethoxysilan, 3-Glycidyloxypropyltriisopropoxysilan.

Es können auch verschiedene Epoxide als Monomere eingesetzt werden, wobei sowohl Gemische als auch sequentielle Dosierungen möglich sind.

Der $CO_2$-Druc für die Umsetzung der Epoxide mit $CO_2$ beträgt beispielsweise $\geq 1$ bar bis $\leq 80$ bar, vorzugsweise $\geq 2$ bar bis $\leq 50$ bar.

Die Reaktionstemperaturen für die Umsetzung der Epoxide mit $CO_2$ betragen beispielsweise $\geq 0$ °C bis $\leq 150$ °C, vorzugsweise $\geq 10$ °C bis $\leq 130$ °C. Aufgrund der vergleichsweise niedrigen möglichen Reaktionstemperaturen ist es besser als bei Prozessen nach dem Stand der Technik möglich, Epoxide mit thermolabilen Funktionalitäten wie Alkoxysilanen oder Doppelbindungen als Co-Monomere einzusetzen, ohne dass es zu unerwünschten Reaktionen, die zur Vernetzung führen können, kommt.

Die Reaktion kann absatzweise, oder in einem halbkontinuierlichen oder kontinuierlichen Verfahren durchgeführt werden, wobei die Reaktanden in beliebiger Reihenfolge dosiert werden können.

Es können auch Gemische von Komplexen mit verschiedenen Liganden der Formel (Ia)/(Ib) und verschiedenen Zentralionen als Katalysator eingesetzt werden.

Falls die Oxidationszahl des Zentralions größer als +II ist, kann die überschüssige Ladung durch ein geeignet gewähltes Anion kompensiert werden. Hierbei kommen beispielsweise Halogenide, Carboxylate, Phenolate, Sulfonate, Phosphonate, Alkylreste, Alkoxyreste oder Amido-Verbindungen in Frage. Bei den Carboxylaten können deren aliphatische oder aromatische Reste durch Fluor substituiert sein. Die Phenolate können durch elektronenziehende Substituenten wie Nitro, Fluor, Chlor, Estergruppen oder Trifluormethyl substituiert sein.

Nicht belegte Koordinationsstellen in den Katalysatorkomplexen können durch Lösungsmittelmoleküle oder zusätzliche Liganden, wie Pyridin, Acetonitril, Tetrahydrofuran, als Starter verwendete Alkohole, dem entstehenden Polycarbonat, oder dem Co-Katalysator abgesättigt werden.

Die Reaktion kann in Abwesenheit oder in Gegenwart von Lösungsmitteln, wie beispielsweise in Ethern, in cyclischen Ethern, wie Tetrahydrofuran oder Dioxan, in halogenierten Kohlenwasserstoffen, wie Dichlormethan, Dichlorethan, Dichlorethylen, Chlorbenzol, oder Dichlorbenzol in Aromaten, wie Benzol oder Toluol, in Nitrilen, wie Acetonitril oder in dipolar aprotischen Lösungsmitteln, wie Dimethylformamid, *N*-Methyl-2-pyrrolidon, Triethylphosphat oder Dimethylsulfoxid durchgeführt werden.

Die erfindungsgemäßen Komplexe sind technisch einfach zugänglich, die erfindungsgemäß verwendeten Liganden ermöglichen die einfache Synthese einer Katalysator-Bibliothek mit verschiedenen Zentralionen. Die Reaktion von Epoxiden und $CO_2$ verläuft unter Katalyse mit den erfindungsgemäßen Komplexen schon bei niedrigen Temperaturen.

Die vorliegende Erfindung wird nachfolgend mit weiteren Ausführungsformen und Aspekten beschrieben. Sie können beliebig kombiniert werden, sofern sich aus dem Zusammenhang nicht eindeutig das Gegenteil ergibt.

In einer Ausführungsform des erfindungsgemäßen Verfahrens steht A für $OCOCH_3$, $OCOCF_3$, $OSO_2CF_3$, $OSO_2C_6H_5CH_3$, $OSO_2CF_3$, $OSO(CH_3)_2$ oder Halogenid.

In einer weiteren Ausführungsform des erfindungsgemäßen Verfahrens ist M ausgewählt aus der Gruppe Zn (II), Cr(II), Mn(II), Mg(II), Fe(II), Co(II), Cr(III)-A, Mn(III)-A, Fe(III)-A und/oder Co(III)-A.

In einer weiteren Ausführungsform des erfindungsgemäßen Verfahrens ist der Ligand L ausgewählt aus der Gruppe umfassend Verbindungen der Formeln (IV) bis (VII):

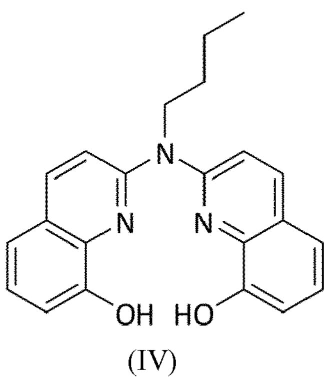

(IV)

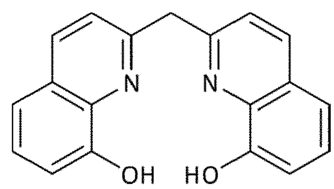

(V)

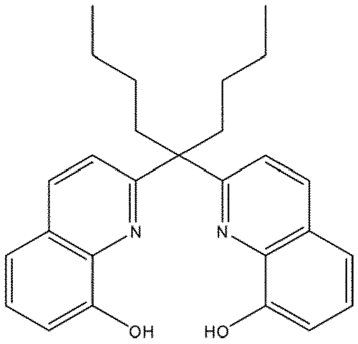

(VI)

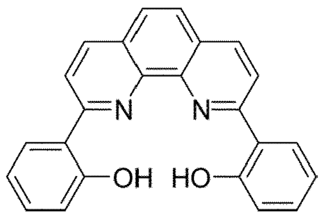

(VII)

In einer weiteren Ausführungsform des erfindungsgemäßen Verfahrens ist der Katalysator ausgewählt aus der Gruppe umfassend Verbindungen der Formeln (VIII) bis (XVIII):

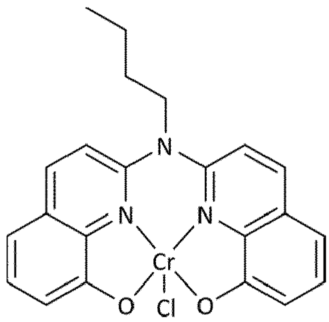

(VIII)

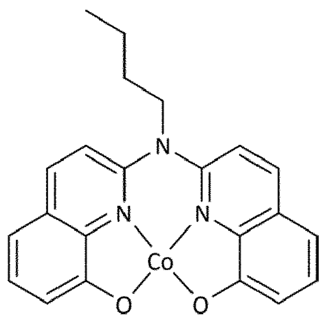

(IX)

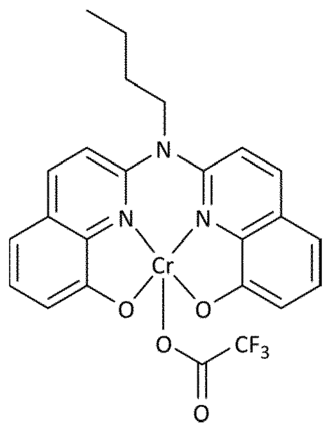

(X)

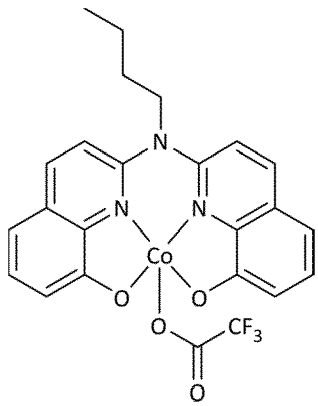

(XI)

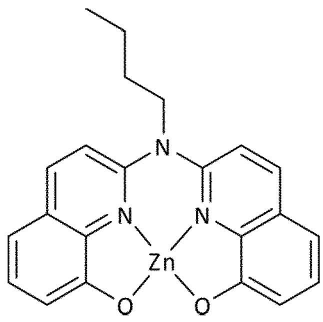

(XII)

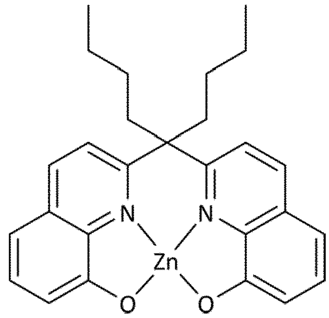

(XIII) (XIV) (XV)

(XVI) (XVII) (XVIII)

In einer weiteren Ausführungsform des erfindungsgemäßen Verfahrens werden als Epoxid Ethylenoxid, Propylenoxid, Cyclohexenoxid oder Styroloxid oder beliebige Mischungen umfassend Ethylenoxid, Propylenoxid, Cyclohexenoxid und Styroloxid eingesetzt.

In einer weiteren Ausführungsform des erfindungsgemäßen Verfahrens wird die Reaktion bei einer Temperatur von ≥ 60 °C bis ≤ 120 °C durchgeführt. Vorzugsweise beträgt die Reaktionstemperatur ≥ 60 °C bis ≤ 100 °C.

In einer weiteren Ausführungsform des erfindungsgemäßen Verfahrens wird die Katalysator enthaltende Reaktionsmischung vor dem Kontaktieren mit Epoxid auf eine Temperatur von ≥ 40 °C bis ≤ 150 0°C erwärmt. Ebenfalls ist es möglich, die Katalysator enthaltende Reaktionsmischung mit einer Teilmenge des Epoxids zu versetzen und auf eine Temperatur von ≥ 40 °C bis ≤ 150 °C zu erwärmen.

Diese Aktivierung des Katalysators kann unter Vakuum, $CO_2$ oder einem Inertgas, wie Stickstoff oder Argon erfolgen. Die Drücke bei der Aktivierung betragen in der Regel ≥ 0,001 bar bis ≤100 bar, vorzugsweise ≥ 0,01 bar bis ≤ 50 bar. Es ist auch möglich, die Aktivierung in Gegenwart von als Starter verwendeten Alkoholen (s. unten) und/oder Epoxiden durchzuführen, wobei diese auch im Laufe der Aktivierung zudosiert werden können.

Vorzugsweise wird nach der Aktivierung die Temperatur auf unter 80 °C gesenkt, bevor die eigentliche Reaktion beginnt.

In einem alternativen Verfahren wird die anschließende Umsetzung von Epoxiden und $CO_2$ bei Temperaturen von 0 bis 150 °C, vorzugsweise 10 bis 100 °C, durchgeführt.

In einer weiteren Ausführungsform des erfindungsgemäßen Verfahrens wird weiterhin ein Co-Katalysator aus der Gruppe der Ammoniumsalze, Phosphoniumsalze, Bis(triphenylphosphin)-iminiumsalze, Amidine, Guanidine oder DMAP (4-(Dimethylamino)-pyridin) in der Reaktion eingesetzt.

Solche Co-Katalysatoren können beispielsweise in Mengen von ≥ 0,1 Mol bis ≤10 Mol, bezogen auf die Anzahl der eingesetzten Mole an Katalysator, zugesetzt werden.

Besonders bevorzugte Co-Katalysatoren sind PPNC1 (Bis(triphenylphosphoranylidene)ammoniumchlorid), TBACl (Tetrabutylammoniumchlorid), und/oder TBABr (Tetrabutylammoniumbromid).

Bevorzugte Guanidine sind DBU (1,8-Diazabicyclo[5.4.0]undec-7-en), DBN (1,5-Diazabicyclo[4.3.0]non-5-en) oder TBD (1,5,7-Triazabicyclo[4.4.0]dec-5-en).

In einer weiteren Ausführungsform des erfindungsgemäßen Verfahrens wird die Reaktion in Gegenwart eines Alkohols mit ≥ 1 bis ≤ 8 OH-Gruppen durchgeführt.

Solche als Starter verwendete Alkohole können Molgewichte von ≥ 31 g/mol bis ≤ 2000 g/mol aufweisen. Der Rest kann aliphatisch, aromatisch, cycloaliphatisch oder araliphatisch sein oder Ethergruppen enthalten. Bevorzugt sind Octandiol (insbesondere 1,8-Octandiol), Decandiol, Dodecandiol, Dimerfettdiol, Polypropylenglykole oder Polytetrahydrofurane. Starter werden vorzugsweise bei der Aktivierung vorgelegt, sie können aber auch während der Aktivierung oder zu Beginn der eigentlichen Reaktion zu dosiert werden.

Ein weiterer Gegenstand der vorliegenden Erfindung ist ein polymerer Carbonatester, erhältlich durch die Reaktion eines cyclischen Epoxids mit Kohlendioxid in einem erfindungsgemäßen Verfahren, wobei das zahlenmittlere Molekulargewicht $M_n$, bestimmt mittels Gelpermeationschromatographie, in einem Bereich von ≥ 500 g/mol bis ≤ 50000 g/mol, bevorzugt in einem Bereich von ≤ 500 g/mol bis ≤ 5000 g/mol liegt und der Polydispersitätsindex $M_w/M_n$ in einem Bereich von ≥ 1,0 bis ≤ 1,5, bevorzugt ≥ 1,1 bis ≤ 1,4 liegt. Das Molekulargewicht kann gemäß DIN 55672-1 mit THF als Elutionsmittel gegen Polystyrol-Standards bestimmt werden.

Vorzugsweise ist hierbei das cyclische Epoxid Cyclohexenoxid.

Die vorliegende Erfindung betrifft ebenfalls die Verwendung von Komplexen eines Metalls M oder M-A mit einem Liganden L als Katalysatoren für die Reaktion eines Epoxids mit Kohlendioxid, wobei das Metall M in einer Oxidationsstufe von ≥ 0 vorliegt,

A für Halogenid, Carboxylat, Phenolat, Sulfonat, Phosphonat, Alkyl, Alkoxy oder Amido steht und der Ligand L die nachfolgende Struktur (Ia) oder (Ib) aufweist, wobei weiterhin eine oder beide der in (Ia) und/oder (Ib) gezeigten OH-Gruppen deprotoniert sein können:

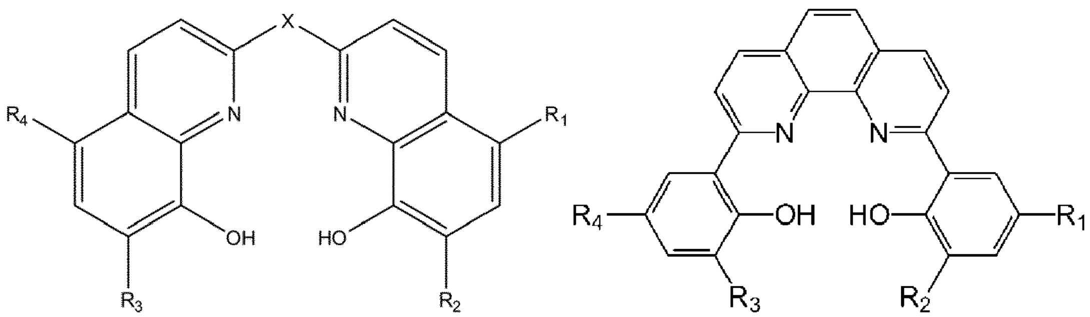

(Ia) (Ib)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen und X für eine Brücke der Formel (II) oder (III) steht:

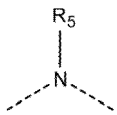 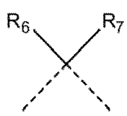

(II) (III)

wobei R5 für einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest, oder einen gegebenenfalls Alkyl-substituierten Pyridylrest oder Pyridylmethylrest steht und R6 und R7 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest oder einen -COOR8-Rest stehen, wobei R8 für einen $C_1$-$C_8$-Alkylrest steht.

Bevorzugte Ausführungsformen der Liganden und Katalysatoren, welche in Bezug auf das erfindungsgemäße Verfahren erläutert wurden, sind in der erfindungsgemäßen Verwendung selbstverständlich ebenfalls mit eingeschlossen.

Ein weiterer Aspekt der vorliegenden Erfindung sind Metallkomplexe gemäß der Formel (XIX), bei denen das Metall M Cr(III)-A, Co(III)-A und/oder Zn(II) ist und A für Halogenid, Carboxylat, Phenolat, Sulfonat, Phosphonat, Alkyl, Alkoxy oder Amido steht

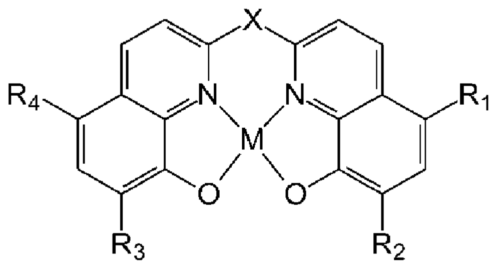

(XIX)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen und X für eine Brücke der Formel (II) oder (III) steht:

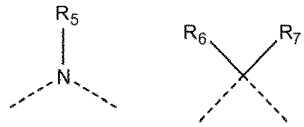

(II) (III)

wobei R5 für einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest, oder einen gegebenenfalls Alkyl-substituierten Pyridylrest oder Pyridylmethylrest steht und
R6 und R7 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest oder einen -COOR8-Rest stehen, wobei R8 für einen $C_1$-$C_8$-Alkylrest steht;
oder Metallkomplexe der Formel gemäß der Formel (XX), bei denen das Metall M Cr(III)-A, Co(III)-A und/oder Zn(II) ist und A für Halogenid, Carboxylat, Phenolat, Sulfonat, Phosphonat, Alkyl, Alkoxy oder Amido steht:

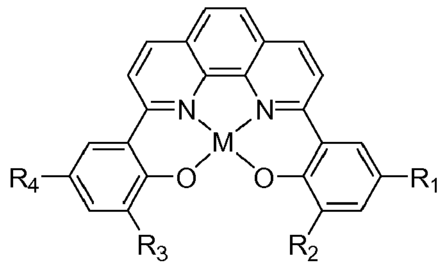

(XX)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen.

Vorzugsweise sind diese Metallkomplexe ausgewählt aus der Gruppe umfassend Verbindungen der Formeln (XXI) bis (XXVI):

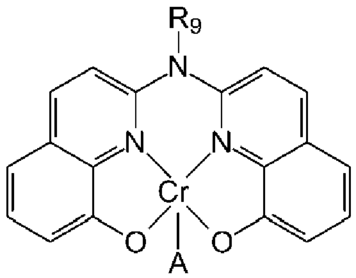
(XXI)

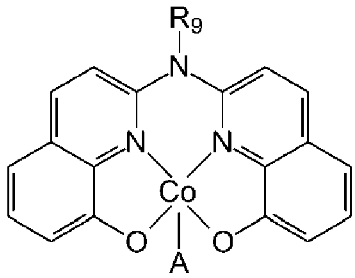
(XXII)

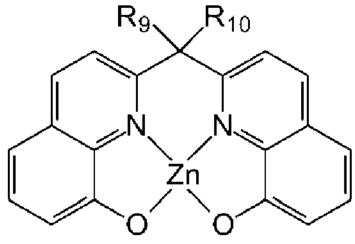
(XXIII)

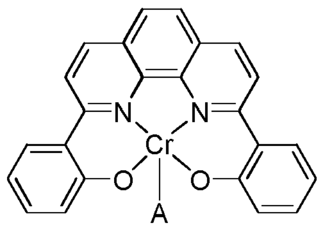
(XXIV)

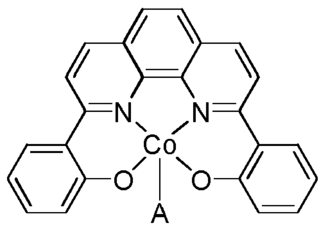
(XXV)

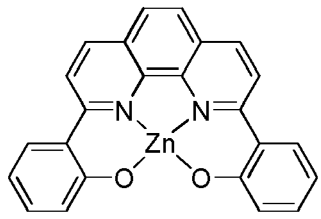
(XXVI)

wobei R9 und R10 unabhängig voneinander für Wasserstoff, Methyl, Ethyl, Propyl, Butyl, Pentyl oder Hexyl stehen und A für $OCOCH_3$, $OCOCF_3$, $OSO_2CF_3$, $OSO_2C_6H_5CH_3$, $OSO_2CF_3$, $OSO(CH_3)_2$ oder Halogenid steht.

**Beispiele**

Die Erfindung wird anhand der nachfolgenden Figuren und Beispiele näher erläutert, ohne jedoch darauf beschränkt zu sein.

Die Angaben zum Druck beziehen sich auf den absoluten Druck.

Der in den Beispielen eingesetzte 160 ml Druckreaktor hatte eine Höhe (innen) von 5,08 cm und einen Innendurchmesser von 6,35 cm. Der Reaktor war ausgestattet mit einem elektrischen Heizmantel (525 Watt maximale Heizleistung). Die Gegenkühlung bestand in einem spiralförmig gebogenen Tauchrohr mit 3,17 mm Außendurchmesser, das bis 5 mm über den Boden in den Reaktor ragte und das mit Kühlwasser von ca. 10 °C durchströmt wurde. Der Wasserstrom wurde über ein Magnetventil ein- und ausgeschaltet. Weiterhin war der Reaktor mit einem Einleitrohr sowie einem Thermofühler mit 1,6 mm Durchmesser ausgestattet, die bis 3 mm über den Boden in den Reaktor ragten.

Bei dem in den Beispielen eingesetzten Hohlwellenrührer handelte es sich um einen Hohlwellenrührer, bei dem das Gas über eine hohle Welle des Rührers in die Reaktionsmischung eingeleitet wurde. Der auf der Hohlwelle angebrachte Rührkörper wies vier Arme auf mit einem Durchmesser von 50 mm und eine Höhe von 20 mm. An jedem Ende des Arms war ein Gasauslass angebracht, der einen Durchmesser von 3 mm aufwies. Durch die Drehung des Rührers entstand ein Unterdruck derart, dass das über der Reaktionsmischung befindliche Gas ($CO_2$ und ggf. Alkylenoxid) abgesaugt wurde und über die Hohlwelle des Rührers in die Reaktionsmischung eingeleitet wurde.

Für die Aufnahme der Epoxid-Konzentration und die Bildung der Produkte während der Umsetzung von Epoxiden und $CO_2$ wurde ein Bruker MATRIX-MF Spektrometer ausgestattet mit einer 3,17 mm ATR-IR Faseroptik-Sonde verwendet. Die ATR-IR Faseroptik-Sonde (90° Diamant-Prisma mit 1 $\times$ 2 mm Grundfläche und 1 mm Höhe als ATR Element, 2 x 45° Reflektion des IR Strahls, IR Strahl über Lichtleitfaser eingekoppelt) wurde so in den Reaktor eingebaut, dass der am Ende der 3,17 mm ATR Faseroptik-Sonde befindliche Diamant vollständig in die Reaktionsmischung eintauchte. IR Spektren (Mittelwert von 100 Scans) wurden zeitaufgelöst im Bereich 4000-400 $cm^{-1}$ mit einer Auflösung von 4 $cm^{-1}$ aufgenommen. Die erhaltenden Spektren wurden unter Einsatz der Software PEAXACT ausgewertet. Die Propylenoxid-Konzentration wurde über Aufnahme der charakteristischen Bande für Propylenoxid bei 830 $cm^{-1}$ verfolgt. Die Konzentration von cyclischem Propylencarbonat wurde über Aufnahme der charakteristischen Bande für cyclisches Propylencarbonat bei 1810 $cm^{-1}$ verfolgt. Die Cyclohexenoxid-Konzentration wurde über Aufnahme der charakteristischen Bande für Cyclohexenoxid bei 815 $cm^{-1}$ verfolgt. Die Konzentration von cyclischem Cyclohexencarbonat wurde über Aufnahme der charakteristischen Bande für cyclisches Cyclohexencarbonat bei 1820 $cm^{-1}$ (cis-Isomeres) und 1804 $cm^{-1}$ (*trans*-Isomeres) verfolgt. Die Konzentration von Polycyclohexencarbonat wurde über Aufnahme der charakteristischen Bande für Polycyclohexencarbonat bei 1743 $cm^{-1}$ verfolgt.

Die Charakterisierung des Reaktionsgemisches erfolgte durch $^1$H-NMR Spektroskopie und Gelpermeations-

chromatographie.

Bei der Umsetzung von Propylenoxid und $CO_2$ resultierte in den vorliegenden Beispielen das cyclische Propylencarbonat. Der molare Anteil des umgesetzten Propylenoxids (C in mol%) wurden mittels $^1$H-NMR Spektroskopie bestimmt. Die Probe wurde jeweils in deuteriertem Chloroform gelöst und auf einem Spektrometer der Firma Bruker (AV400, 400 MHz) vermessen. Die relevanten Resonanzen in den $^1$H-NMR Spektren (bezogen auf TMS = 0 ppm), die zur Integration verwendet wurden, sind wie folgt:

I1: 1,45 - 1,49: Methylgruppe des cyclischen Carbonats, Fläche der Resonanz entspricht drei H Atomen,

I2: 2,95 - 2,99: Methingruppe für freies, nicht abreagiertes Propylenoxid, Fläche der Resonanz entspricht einem H Atom.

Unter Berücksichtigung der relativen Intensitäten wurden die Werte wie folgt berechnet: Der molare Anteil des umgesetzten Propylenoxids (C in mol%) bezogen auf die Summe der bei der Aktivierung und der Copolymerisation eingesetzten Menge an Propylenoxid, berechnet sich nach der Formel (XXVII):

$$C = [(I1/3) / ((I1/3) + (I2/1))] \times 100\% \qquad \text{(XXVII)}$$

Bei der Copolymerisation von Cyclohexenoxid und $CO_2$ resultierte neben dem cyclischen *cis*- und *trans*-Cyclohexencarbonat das Polycyclohexencarbonat, welches die in Formel (XXVIII) gezeigte Polycarbonat-Einheiten enthält.

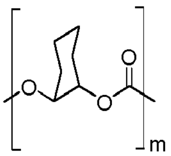

(XXVIII)

Das Verhältnis der Menge an cyclischem Cyclohexencarbonat zu Polycyclohexencarbonat (Selektivität c/1) sowie der molare Anteil des umgesetzten Cyclohexenoxids (C in mol%) wurden mittels $^1$H-NMR Spektroskopie bestimmt. Die Probe wurde jeweils in deuteriertem Chloroform gelöst und auf einem Spektrometer der Firma Bruker (AV400, 400 MHz) vermessen. Die relevanten Resonanzen in den $^1$H-NMR Spektren (bezogen auf TMS = 0 ppm), die zur Integration verwendet wurden, sind wie folgt:

I3: 4,51: Methingruppe der Polycarbonat-Einheiten, Fläche der Resonanz entspricht zwei H Atomen,

I4: 3,81: Methingruppe des cyclischen Carbonats, Fläche der Resonanz entspricht zwei H Atomen,

I5: 2,98: Methingruppe für freies, nicht abreagiertes Cyclohexenoxid, Fläche der Resonanz entspricht zwei H Atomen.

Angegeben sind das molare Verhältnis der Menge an cyclischem Cyclohexencarbonat zu Carbonat-Einheiten im Polycyclohexencarbonat (Selektivität c/1) sowie der molare Anteil des umgesetzten Cyclohexenoxids (C in mol%).

Unter Berücksichtigung der relativen Intensitäten wurden die Werte wie folgt berechnet:

Molares Verhältnis der Menge an cyclischem Cyclohexencarbonat zu Carbonat-Einheiten im Polycyclohexencarbonat (Selektivität c/1):

$$c/l = I4 / I3 \qquad \text{(XXIX)}$$

Der molare Anteil des umgesetzten Cyclohexenoxids (C in mol%) bezogen auf die Summe der bei der Aktivierung und der Copolymerisation eingesetzten Menge an Cyclohexenoxid, berechnet sich nach der Formel:

$$C = [((I3/2) + (I4/2)) / ((I3/2) + (I4/2) + (I5/2))] \times 100\% \qquad \text{(XXX)}$$

Das Zahlenmittel $M_n$ und das Gewichtsmittel $M_w$ des Molekulargewichts der entstandenen Polymere wurde mittels Gelpermeationschromatographie (GPC) bestimmt. Es wurde vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei wurden Polystyrolproben bekannter Molmasse der Fa. PSS Polymer Service zur Kalibrierung verwendet.

**Beispiel 1: Ligand $H_2$(babhq)**

Der Ligand $H_2$(babhq) der Formel (XXXI) wurde von Sigma-Aldrich bezogen (CAS 82361-90-8).

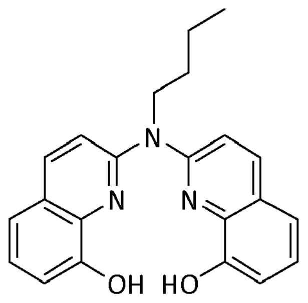

(XXXI)

**Beispiel 2: Synthese des Liganden $H_2$(bpphen)**

Der Ligand $H_2$(bpphen) der Formel (XXXII) wurde in drei Stufen ausgehend von Anisol (CAS 100-66-3) hergestellt. Die in der Formel (XXXII) angegebenen Zahlen bezeichnen dabei die Nummerierung der Atome für die Zuordnung der Signale in den NMR-Spektren. Die Produkte der Stufen eins bis drei (2-Lithioanisol CAS 31600-86-9, 2,9-Bis (2-methoxyphenyl)-1,10-phenanthrolin CAS 127347-71-1, $H_2$(bpphen) CAS 192631-69-9) sind literaturbekannt und wurden in Anlehnung an S. Routier, V. Joanny, A. Zaparucha, H. Vezin, J.-P. Catteau, J.-L. Bernier, C. Bailly, *J. Chem. Soc.*, *Perkin Trans.2* **1998**, 863-868 wie folgt synthetisiert:

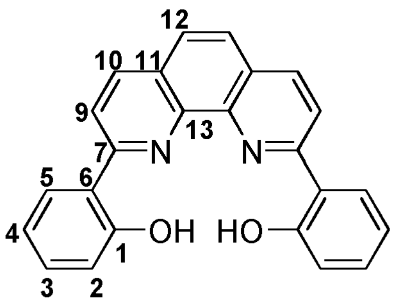

(XXXII)

In der ersten Stufe wurde 2-Lithioanisol durch Umsetzung von 2-Bromanisol mit Butyllithium gemäß Formel (XXXIII) hergestellt:

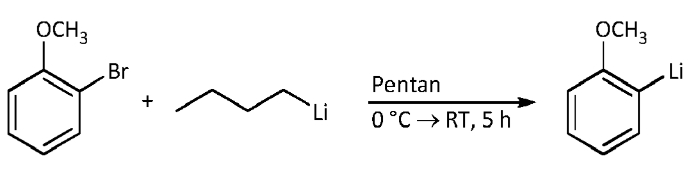


(XXXIII)

2-Bromanisol (45,6 g, 244 mmol, 1,0 eq.) wurde in Pentan (100 mL) gelöst und die Lösung auf 0 °C gekühlt. Unter starkem Rühren wurde *n*-Butyllithium (160 mL, 1,6 M in Hexan, 256 mmol, 1,1 eq.) langsam zugetropft. Es bildete sich eine weiße Suspension, die 30 min bei 0 °C und anschließend 2 h bei Raumtemperatur gerührt wurde. Der weiße Feststoff wurde abgetrennt, mit Pentan (30 mL) gewaschen und unter Feinvakuum getrocknet.

Ausbeute: 27,0 g (237 mmol, 97%), weißes Pulver.

In der zweiten Stufe wurde 1,10-Phentanthrolin mit dem 2-Lithioanisol gemäß Formel (XXXIV) in einer Tschit-

schibabin-analogen Reaktion zu $Me_2$(bpphen) umgesetzt:

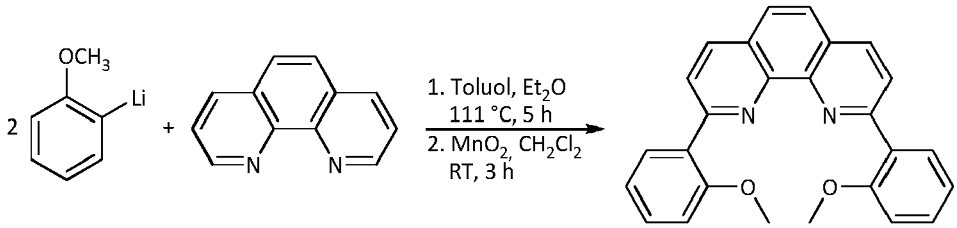


(XXXIV)

Eine Lösung von 1,10-Phenanthrolin-Monohydrat (4,30 g, 21,7 mmol, 1,0 eq.) in Toluol (150 mL) wurde tropfenweise mit einer Lösung von 2-Lithioanisol (15,0 g, 131,5 mmol, 6,1 eq.) aus Stufe eins in Diethylether (80 mL) versetzt. Hierbei wurde die Reaktionsmischung erst gelb, dann grünlich und schließlich intensiv rot. Zunächst wurde der Diethylether abdestilliert, anschließend wurde für 5 h unter Rückfluss erhitzt. Anschließend wurde die Reaktionslösung durch Zugabe von Wasser (100 mL) bei Raumtemperatur gequencht, die organische Phase abgetrennt und die wässrige Phase viermal mit Dichlormethan (á 50 mL) extrahiert. Die vereinigten organischen Phasen wurden etwas eingeengt und für drei Stunden über aktiviertem Braunstein (180 g, 2,1 mol, 97 eq. MERCK) gerührt. Die Reaktionsmischung wurde danach mit Natriumsulfat getrocknet und über Celite® filtriert. Das Filtrat wurde vollständig eingeengt und das Nebenprodukt Anisol bei 80 °C am Feinvakuum abkondensiert.

Ausbeute: 7,61 g (19,4 mmol, 89%), gelber Feststoff.

HR-ESI/MS ($CH_3OH$): m/z berechnet für $[C_{26}H_{20}N_2O_2+H]^+$: 393,1598, gefunden: 393,1587; m/z berechnet für $[C_{26}H_{20}N_2O_2+Na]^+$: 415,1417, gefunden: 415,1419.

$^1$H-NMR (300,1 MHz, $CDCl_3$): δ (ppm) = 3,82 (s, 6H, $OCH_3$), 6,96 (d, *J* = 8,2 Hz, 2H, $H_{Ar}$), 7,11 (dt, *J* = 7,5, 0,9 Hz, 2H, $H_{Ar}$), 7,31-7,39 (m, 2H, $H_{Ar}$), 7,71 (s, 2H, $H_{Ar}$), 8,11-8,19 (m, 4H, $H_{Ar}$), 8,23 (dd, *J* = 7,6, 1,8 Hz, 2H, $H_{Ar}$).

$^{13}$C{$^1$H}-NMR (75,5 MHz, $CDCl_3$): δ (ppm) = 55,8 ($OCH_3$), 111,6 ($C_{Ar}$), 121,4 ($C_{Ar}$), 124,8 ($C_{Ar}$), 125,9 ($C_{Ar}$), 127,4 ($C_{Ar}$), 129,9 ($C_{Ar}$), 130,2 ($C_{Ar}$), 132,5 ($C_{Ar}$), 135,2 ($C_{Ar}$), 146,3 ($C_{Ar}$), 156,3 ($C_{Ar}$), 157,6 ($C_{Ar}$).

In der dritten Stufe wurde gemäß Formel (XXXV) mit Natriumethanthiolat entschützt:

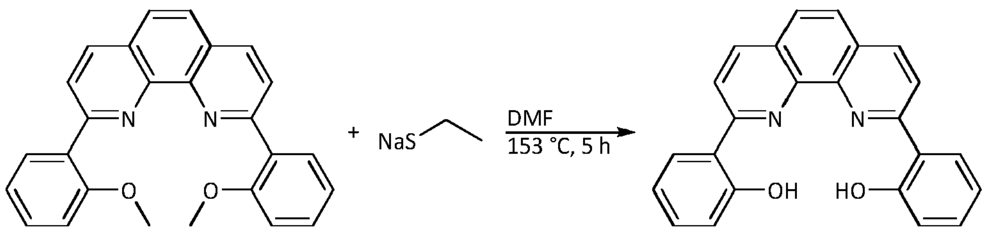


(XXXV)

Eine rote Lösung von $Me_2$(bpphen) (7,61 g, 19,4 mmol, 1,0 eq.) aus Stufe zwei in Dimethylformamid (230 mL) wurde mit Natriumethanthiolat (8,37 g, 99,5 mmol, 1,0 eq., hergestellt aus Ethanthiol und Natriumhydrid) versetzt, wobei diese braun wurde. Die Reaktionsmischung wurde im Anschluss 5 h unter Rückfluss erhitzt, abgekühlt und unter Eiskühlung mit einem 1:1-Gemisch aus 10 %iger Natronlauge und 20 %iger WasserstoffperoxidLösung versetzt, bis keine Gasentwicklung mehr beobachtet werden konnte. Nach Rühren bei Raumtemperatur über Nacht wurde die gelbe Mischung mit 20 %iger Schwefelsäure auf pH = 7 eingestellt. Hierbei fiel ein gelber Feststoff aus, der abfiltriert, mit Wasser gewaschen und aus Ethanol umkristallisiert wurde. Um Verunreinigungen durch Salzverbindungen abzutrennen, wurde der erhaltene Feststoff mit Dichlormethan extrahiert und wieder eingeengt und am Feinvakuum getrocknet.

Ausbeute: 4,27 g (11,7 mmol, 60%), gelbe Nadeln.

HR-ESI/MS ($CH_3OH$): m/z berechnet für $[C_{24}H_{16}N_2O_2+H]^+$: 365,1285, gefunden: 365,1282; m/z berechnet für $[C_{24}H_{16}N_2O_2+Na]^+$: 387,1104, gefunden: 387,1108.

$^1$H-NMR (300,1 MHz, $CDCl_3$): δ (ppm) = 6,98-7,06 (m, 2H, H4), 7,09 (dd, *J* = 8,2, 1,0 Hz, 2H, H2), 7,41 (ddd, *J* = 8,5, 7,3, 1,5 Hz, 2H, H3), 8,03 (s, 2H, H12), 8,25 (dd, *J* = 8,0, 1,5 Hz, 2H, H5), 8,56 (d, *J*= 8,8 Hz, 2H, H9), 8,65 (d, *J*= 8,7 Hz, 2H, H10), 13,94 (s, 2H, OH).

$^{13}$C{$^1$H}-NMR (75,5 MHz, $CDCl_3$): δ (ppm) = 117,8 (C2), 119,1 (C4), 120,3 (C6), 121,3 (C9), 126,2 (C12), 127,3 (C11), 128,6 (C5), 131,9 (C3), 138,2 (C10), 141,5 (C13), 157,1 (C7), 159,1 (C1).

**Beispiel 3: Synthese des Liganden $H_2$(nbhq)**

Die Zahlen bezeichnen die Nummerierung für die Zuordnung der Signale in den NMR-Spektren.

Der Ligand $H_2$(nbhq) der Formel (XXXVI) wurde in sechs Stufen ausgehend von 8-Hydroxychinolin (CAS 148-24-3) hergestellt. Die Produkte der Stufen eins bis vier (1-Oxy-8-hydroxychinolin CAS 1127-45-3, Dimethyl-2,2-bis (8-acetoxychinolin-2-yl)malonat CAS 69618-69-5, $H_2$(mbhq) CAS 63969-39-1, [Ni(mbhq)] CAS 69595-98-8) sind literaturbekannt und wurden in Anlehnung an Y. Yamamoto, A. Miura, A. Kawamata, M. Miura, S. Takei, *Bull. Chem. Soc. Jpn.* **1978**, *51*, 3489-3495 wie folgt synthetisiert:

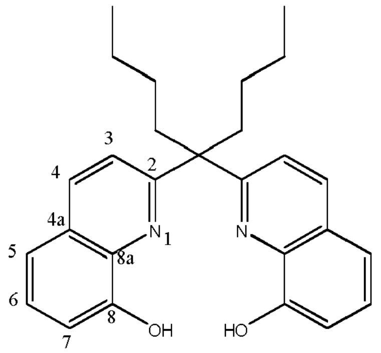

(XXXVI)

In der ersten Stufe wurde 8-Hydroxychinolin gemäß Formel (XXXVII) mit Peroxyessigsäure oxidiert:

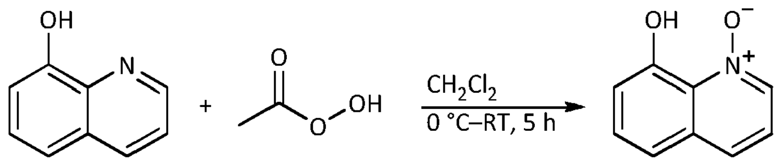


(XXXVII)

Zu einer Lösung aus 8-Hydroxychinolin (50,0 g, 344 mmol) in Methylenchlorid (325 mL) wurden bei 0 °C verdünnte Peroxyessigsäure (Fluka, ~39 % in wässriger Essigsäure, 75,1 mL, 440 mol) tropfenweise zugegeben, wobei die gelbe Lösung tiefrot wurde. Nach Erwärmen und Rühren bei Raumtemperatur für 5 h wurde eine Lösung aus Natriumdisulfit (14,0 g, 74 mmol) in Wasser (20 mL) tropfenweise zugegeben. Hierbei färbte sich die Lösung dunkelgelb bis schwarz. Die organische Phase wurde nacheinander mit 1 M Salzsäure (ca. 300 mL), ges. Natriumhydrogencarbonat-Lösung (ca. 200 mL), ges. Kaliumcarbonat-Lösung (ca. 150 mL) und ges. Natriumchlorid-Lösung. (ca. 200 mL) gewaschen. Die gelb-braune Lösung wurde mit Kieselgel (Kieselgel 60 für die Säulenchromatographie, 0,063-0,200 mm, 5 g) und zum Trocknen mit Natriumsulfat versetzt. Nach Rühren bei Raumtemperatur für 15 min wurde der Feststoff abgetrennt und das Lösungsmittel am Rotationsverdampfer entfernt. Es wurde ein gelber bis leicht bräunlicher Feststoff erhalten, der unter Feinvakuum getrocknet wurde.

Ausbeute: 42,4 g (263 mmol, 76%), gelber Feststoff.

HR-ESI/MS ($CH_3OH$): m/z berechnet für $[C_9H_7NO_2+H]^+$: 162,0550, gefunden: 162,0553; m/z berechnet für $[C_9H_7NO_2+Na]^+$: 184,0374, gefunden: 184,0374.

$^1$H-NMR (300,1 MHz, $CDCl_3$): $\delta$ (ppm) = 7,09 (dd, *J* = 7,9, 1,1 Hz, 1H, H7), 7,24-7,29 (m, 2H, H3/H5), 7,51 (t, *J* = 7,9 Hz, 1H, H6), 7,81 (dd, *J* = 8,5, 0,6 Hz, 1H, H4), 8,26 (dd, *J* = 6,1, 1,0 Hz, 1H, H2), 15,09 (s, 1H, OH).

$^{13}C\{^1H\}$-NMR (75,5 MHz, $CDCl_3$): $\delta$ (ppm) = 114,6 (C7), 116,6 (C5), 120,3 (C3), 129,4 (C4), 129,7 (C4a), 130,3 (C6), 132,1 (C8a), 134,3 (C2), 153,8 (C8).

CHN Analyse: berechnet für $C_9H_7NO_2$ (161.16 g/mol): C, 67,07; H, 4,38; N, 8,69 wt%; gefunden: C, 67,14; H, 4,19; N, 8,63 wt%.

In der zweiten Stufe folgte die Umsetzung mit Dimethylmalonat gemäß Formel (XXXVIII):

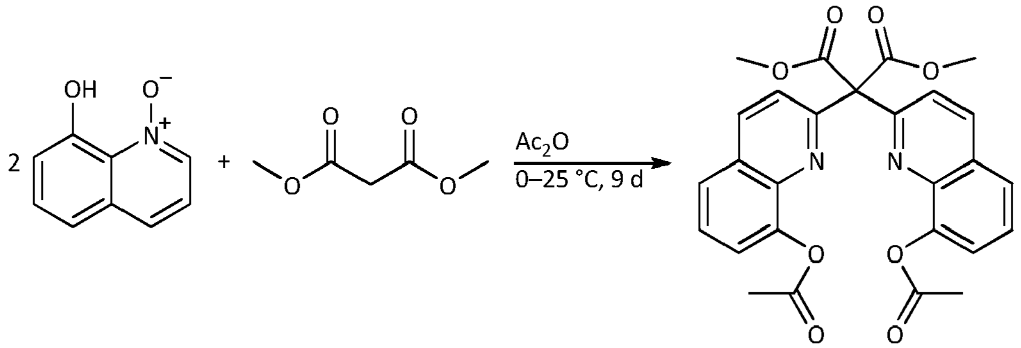


(XXXVIII)

In Essigsäureanhydrid (50 mL) wurden 1-Oxy-8-hydroxychinolin aus Stufe eins (13,78 g, 85,5 mmol, 1.0 eq.) und Dimethylmalonat (10,8 mL, 94.2 mmol, 1.1 eq.) suspendiert und bei 0-25 °C neun Tage lang gerührt. Dabei wurde etwa alle 12 h das Eisbad erneuert, wobei sich die Reaktionsmischung zwischenzeitlich jeweils erwärmte. Nach Zugabe von Methanol (25 mL) wurde die gelbe Suspension bei 0 °C für 5 h gerührt, mit destilliertem Wasser (50 mL) versetzt und bei Raumtemperatur für 20 min gerührt. Der gelbe Niederschlag wurde abgetrennt, mit je 100 mL 50 %iger Essigsäure sowie destilliertem Wasser gewaschen und schließlich unter Feinvakuum getrocknet. Das Rohprodukt wurde ohne weitere Aufreinigung und Charakterisierung weiter umgesetzt.

Ausbeute: 8,46 g, gemäß $^1$H-NMR Spektroskopie 2,2:1 Gemisch von Produkt zu 1-Acetoxychinolon, hellgelbes Pulver.

HR-ESI/MS ($CH_3OH$): m/z berechnet für $[C_{27}H_{22}N_2O_8+Na]^+$: 525,1268, gefunden: 525,1265; m/z berechnet für $[C_{54}H_{44}N_4O_{16}+Na]^+$: 1027,2645, gefunden: 1027,2664.

In Stufe drei wurde gemäß Formel (XXXIX) zum Liganden $H_2$(mbhq) entschützt:

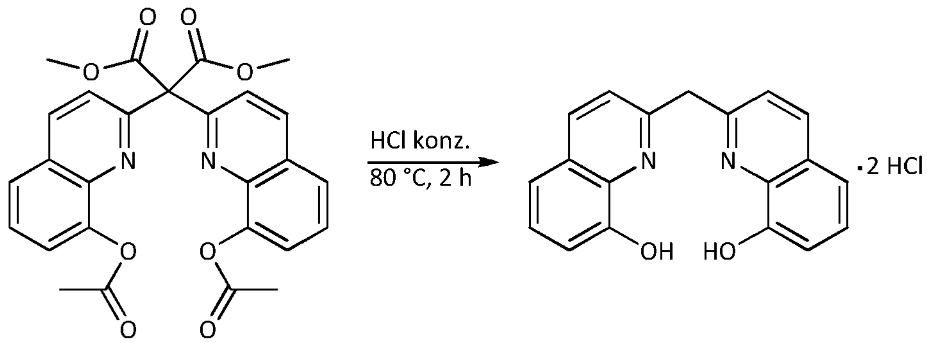


(XXXIX)

Das Produktgemisch aus Stufe zwei wurde in konzentrierter Salzsäure (250 mL) suspendiert und unter Rückfluss für 2 h erhitzt. Der entstandene gelbe Niederschlag wurde anschließend heiß abgetrennt und zweimal mit kalter Salzsäure (20 mL) gewaschen. Das Rohprodukt wurde aus Ethanol (700 mL) umkristallisiert, abfiltriert sowie mit Salzsäure (1 M) und destilliertem Wasser gewaschen und unter Feinvakuum getrocknet.

Ausbeute: 2,83 g (7,57 mmol, 18% über zwei Stufen), gelber bis rötlicher Feststoff.

HR-ESI/MS ($CH_3OH$): m/z berechnet für $[C_{19}H_{14}N_2O_2+H]^+$: 303,1128, gefunden: 303,1129; m/z berechnet für $[C_{19}H_{14}N_2O_2+Na]^+$: 325,0947, gefunden: 325,0944.

$^1$H-NMR (300,1 MHz, $(CD_3)_2SO$): $\delta$ (ppm) = 5,27 (s, 2H, $CH_2$), 7,49-7,55 (m, 2H, H7), 7,58-7,70 (m, 4H, H5/H6), 7,97 (d, *J* = 8,6 Hz, 2H, H3), 8,86 (d, *J* = 8.6 Hz, 2H, H4). Die Alkoholprotonen konnten nicht aufgelöst werden.

$^{13}C\{^1H\}$-NMR (75,5 MHz, $(CD_3)_2SO$): $\delta$ (ppm) = 39,9 ($CH_2$), 115,0 (C6), 117,7 (C5), 123,5 (C3), 128,3 (C4a), 129,9 (C7), 131,7 (C8a), 143,4 (C4), 149,7 (C8), 154,7 (C2).

CHN Analyse: berechnet für $C_{19}H_{14}N_2O_2 \cdot 2HCl$ (374,06 g/mol) C, 60,81; H, 4,30; N, 7,47 wt%; gefunden: C, 60,02; H, 4,62; N, 7,31 wt%.

In der vierten Stufe wurden die Alkoholfunktionen von $H_2$(mbhq) gemäß Formel (XXXX) durch Komplexierung von $Ni^{2+}$ geschützt.

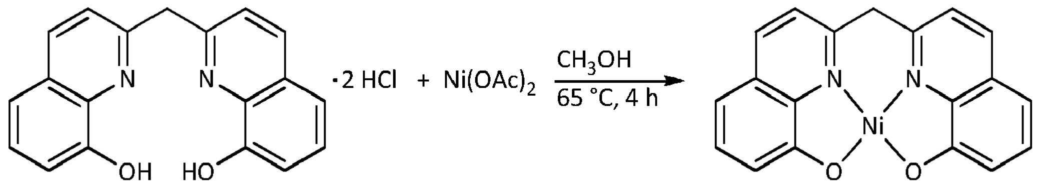


(XXXX)

$H_2$(mbhq) aus Stufe drei (705 mg, 1,88 mmol, 1.0 eq.) wurde in Methanol (20 mL) vorgelegt und mit Nickelacetat-tetrahydrat (580 mg, 2,33 mmol, 1.2 eq.) versetzt. Die Reaktionsmischung wurde im Anschluss unter Rückfluss für vier Stunden erhitzt, wobei sich ein orange-brauner Feststoff bildete. Dieser wurde abgetrennt, mit Methanol (5 mL) gewaschen und bei 50 °C unter Feinvakuum getrocknet.

Ausbeute: 670 mg (1,70 mmol, 90%), orange-brauner Feststoff.

HR-ESI/MS ($CH_3OH$): m/z berechnet für [$C_{19}H_{12}N_2NiO_2$+Nat: 381,0144, gefunden: 381,0147.

$^1$H-NMR/$^{13}$C-NMR: Nicht gemessen, da Substanz schwerlöslich.

CHN Analyse: berechnet für $C_{19}H_{12}N_2NiO_2 \cdot 2H_2O$ (394,05 g/mol): C, 57,77; H, 4,08; N, 7,09 wt%; gefunden: C, 57,54; H, 3,36; N, 7,09 wt%.

In Stufe fünf folgte gemäß Formel (XXXXI) eine Bisalkylierung von [Ni(mbhq)] mit Butyliodid in Gegenwart von Natriumhydroxid:

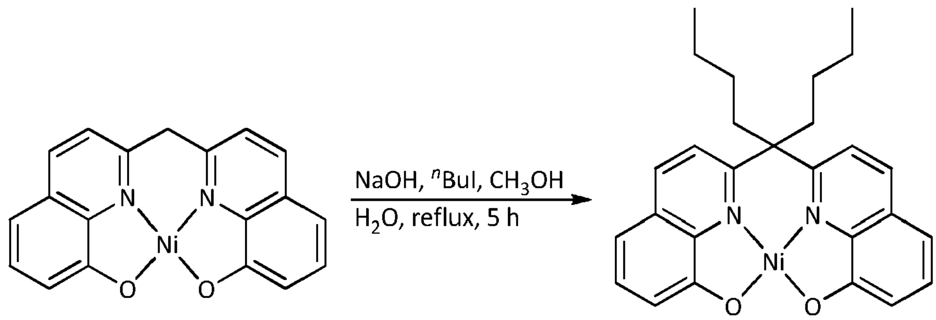


(XXXXI)

Eine Suspension aus [Ni(mbhq)] aus Stufe vier (2,70 g, 7,52 mmol, 1.0 eq.) in 100 ml Methanol wurde bei 50 °C mit Butyliodid (9,0 mL, 76,0 mmol, 10,0 eq.) versetzt. Anschließend wurde eine Lösung aus Natriumhydroxid (0,75 g, 18,8 mmol, 2,6 eq.) in 40 ml entgastem Wasser zugegeben. Die Reaktionslösung wurde unter Rückfluss für fünf Stunden erhitzt und anschließend bei Raumtemperatur für 12 h stehen gelassen. Danach wurde der ausgefallene Feststoff abfiltriert und die Lösung auf Wasser (350 mL) gegeben. Der entstandene Niederschlag wurde ebenfalls abgetrennt, mit dem Ersten vereinigt, mit heißem Wasser gewaschen und unter Feinvakuum getrocknet.

Ausbeute: 3,52 g (7,47 mmol, 99%), brauner Feststoff.

HR-ESI/MS ($CH_3OH$): m/z berechnet für [$C_{27}H_{28}N_2NiO_2$+Na]$^+$: 493,1396, gefunden: 493,1396; m/z berechnet für [$C_{27}H_{27}N_2NiO_2$]$^-$: 469,1431, gefunden: 469,1438.

$^1$H-NMR (300,1 MHz, $(CD_3)_2SO$): δ (ppm) = 0,57 (m, 4H, C*H*$_2$Et), 0,63 (t, *J* = 7,3 Hz, 6H, $CH_3$), 1,03 (td, *J* = 13,9, 7,0 Hz, 4H, C*H*$_2$CH$_3$), 2,44 (m, 4H, C*H*$_2$Pr), 6,78 (d, *J* = 7,6 Hz, 2H, H5), 7,06 (d, *J* = 7,7 Hz, 2H, H7), 7,42 (t, *J* = 7,8 Hz, 2H, H6), 8,16 (d, *J*= 8,7 Hz, 2H, H3), 8,55 (d, *J* = 8,8 Hz, 2H, H4).

$^{13}$C{$^1$H}-NMR (75,5 MHz, $(CD_3)_2SO$): δ (ppm) = 13,3 (*C*H$_2$Et), 21,7 ($CH_3$), 26,3 (*C*H$_2$CH$_3$), 43,2 (*C*H$_2$Pr), 60,7 (*C*Bu$_2$), 110,4 (C7), 113,4 (C5), 123,0 (C3), 127,9 (C4a), 130,5 (C6), 139,2 (C4), 141,8 (C8a), 158,5 (C2), 164,7 (C8).

In Stufe sechs wurde der butylierte Nickelkomplex gemäß Formel (XXXXII) zum Zielliganden $H_2$(nbhq) entschützt:

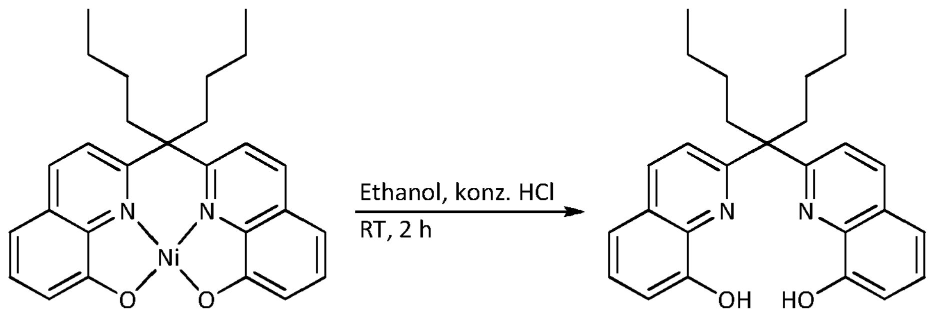


(XXXXII)

Der Nickelkomplex [Ni(nbhq)] aus Stufe fünf (3,32 g, 7,05 mmol, 1,0 eq.) wurde in Ethanol (70 mL) suspendiert und mit konzentrierter Salzsäure (9 mL, 108,72 mmol, 15,4 eq.) versetzt. Diese Mischung wurde bei Raumtemperatur für zwei Stunden gerührt. Der ausgefallene Niederschlag wurde abgetrennt und das Filtrat mit Wasser (150 mL) versetzt. Nach Ruhen bei Raumtemperatur für zwölf Stunden wurde nachgefällter Niederschlag abgetrennt, mit Wasser gewaschen und am Feinvakuum getrocknet.

Ausbeute: 2,40 g (5,78 mmol, 82%), gelbes Pulver.

$^1$H-NMR (300,1 MHz, $(CD_3)_2SO$): $\delta$ (ppm) = 0,73 (t, *J* = 7,3 Hz, 6H, $CH_3$), 0,82-0,92 (m, 4H, $CH_2$Et), 1,16-1,28 (m, 4H, $CH_2CH_3$), 2,59-2,64 (m, 4H, $CH_2$Pr), 7,26 (dd, *J* = 7,4, 1,1 Hz, 2H, H7), 7,44-7,54 (m, 4H, H5/H6), 7,69 (d, *J* = 8,6 Hz, 2H, H3), 8,45 (d, *J* = 8,6 Hz, 2H, H4). Die Alkoholprotonen konnten nicht aufgelöst werden.

$^{13}$C{$^1$H}-NMR (75,5 MHz, $(CD_3)_2SO$): $\delta$ (ppm) = 13,7 ($CH_3$), 22,4 ($CH_2CH_3$), 26,1 ($CH_2$Et), 54,3 ($CH_2$Pr), 55,9 ($CBu_2$), 112,4 (C6), 117,5 (C5), 121,3 (C3), 127,1 (C4a), 127,9 (C7), 134,6 (C8a), 138,7 (C4), 151,9 (C8), 162,7 (C2).

**Beispiel 1-1: Synthese des Chrom-Komplexes [Cr(babhq)OC(O)$CF_3$]**

Der Chrom-Komplex [Cr(babhq)OC(O)$CF_3$] wurde in zwei Stufen synthetisiert. In der ersten Stufe wurde gemäß Formel (XXXXIII) der Komplex [Cr(babhq)Cl] hergestellt.

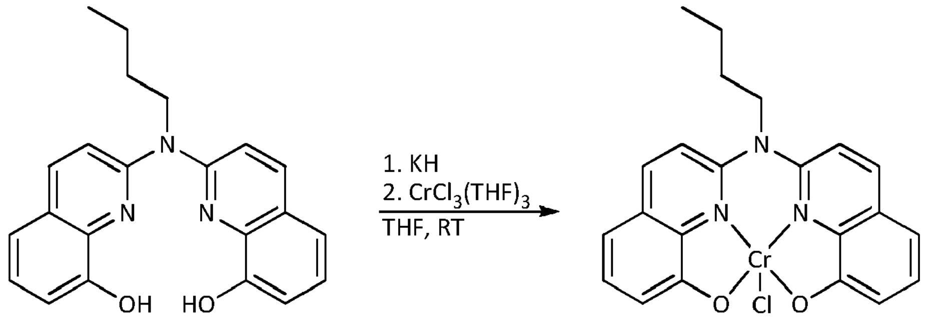


(XXXXIII)

$H_2$(babhq) aus Beispiel 1 (513 mg, 1,43 mmol, 1,0 eq.) und Kaliumhydrid (118 mg, 2,86 mmol, 2,0 eq.) wurden in trockenem THF (20 mL) suspendiert. Diese Mischung wurde gerührt bis die Gasentwicklung aufhörte. Eine Suspension von purpurfarbenen [$CrCl_3(thf)_3$] (537 mg, 1,43 mmol, 1,0 eq.) in trockenem THF (20 mL) wurde hinzugefügt, wobei sich die Reaktionsmischung braun verfärbte. Nach 12-stündigem Rühren bei Raumtemperatur wurde Wasser (100 mL) hinzugefügt. Der gebildete Niederschlag wurde abfiltriert, mit Wasser gewaschen und unter Feinvakuum bei 50 °C getrocknet.

Ausbeute: 545 mg (1,23 mmol, 86%), gelber Feststoff.

HR-ESI/MS ($CH_3OH$): m/z berechnet für $[C_{22}H_{19}CrN_3O_2]^+$: 409,0878, gefunden: 409,0879.

CHN Analyse: berechnet für $C_{22}H_{19}CrN_3O_2 \cdot 2\ H_2O$ (480,88 g/mol): C, 54,95; H, 4,82; N, 9,74 wt%; gefunden: C, 54,84; H, 4,88; N, 9,65 wt%.

Einkristall-Röntgenanalyse: Kristalle der Zusammensetzung [Cr(babhq)Cl(dmf)]·DMF wurden durch Umkristallisieren der Verbindung aus einer gesättigten DMF Lösung, die langsam von 100 °C auf Raumtemperatur gekühlt wurde, erhalten. Eine Einkristall-Röntgenanalyse eines 0,25 × 0,21 × 0,06 mm$^3$ großen Kristalls ergab, dass die Verbindung in der Raumgruppe P $2_1$/n (monoklin) mit *a* = 9,8802(4) Å, *b* = 14,1274(4) Å, *c* = 19,4439(9) Å, $\alpha$ = 90°, $\beta$ = 94,197(3) °, $\gamma$ = 90° und 4 Molekülen je Einheitszelle kristallisiert.

In der zweiten Stufe wurde der Komplex [Cr(babhq)Cl] gemäß Formel (XXXXIV) mit Silbertrifluoracetat umgesetzt.

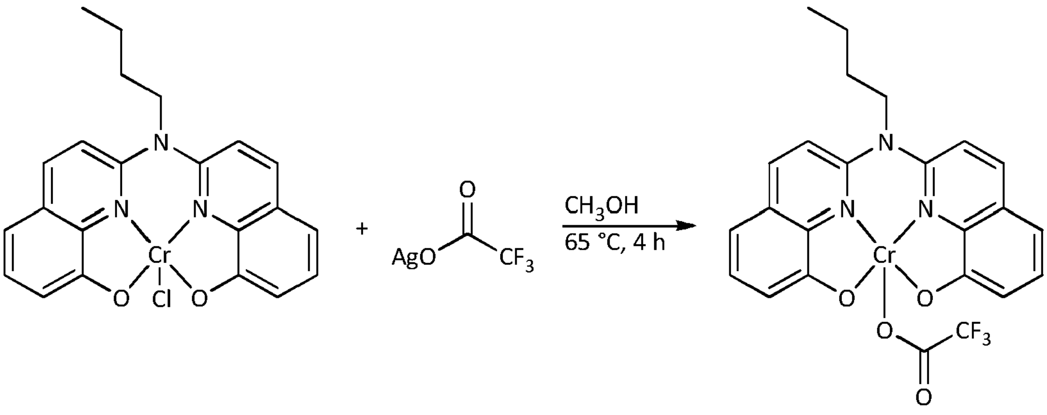


(XXXXIV)

[Cr(babhq)Cl] aus Stufe eins (545 mg, 1,23 mmol, 1,0 eq.) wurde in Ethanol (100 ml) suspendiert. Unter Rühren wurde Silbertrifluoracetat (272 mg, 1,23 mmol, 1,0 eq.) hinzugefügt. Die Mischung wurde unter Rückfluss für 5 h gekocht. Nach Abkühlen auf Raumtemperatur wurden die festen Bestandteile durch Zentrifugieren abgetrennt. Sie wurden mehrmals mit heißem Ethanol extrahiert. Die vereinigten Lösungen wurden zur Trockene eingedampft und unter Feinvakuum bei 50 °C getrocknet.

Ausbeute: 594 mg (1,14 mmol, 92%), brauner Feststoff

HR-ESI/MS ($CH_3CN$): m/z berechnet für $[C_{24}H_{19}CrF_3N_3O_4]^+$: 522,0727, gefunden: 522,0740.

CHN Analyse: $C_{24}H_{19}CrF_3N_3O_4 \cdot EtOH$ (568,12 g/mol) berechnet: C, 54,93; H, 4,43; N, 7,39 wt%; gefunden: C, 54,77; H, 4,46; N, 7,43 wt%.

Einkristall-Röntgenanalyse: Kristalle der Zusammensetzung [Cr(babhq)OC(O)$CF_3$(dmf)] wurden durch Umkristallisieren der Verbindung aus DMF/Ether bei Raumtemperatur als orange Prismen erhalten. Eine Einkristall-Röntgenanalyse eines 0,15 × 0,09 × 0,03 $mm^3$ großen Kristalls ergab, dass die Verbindung in der Raumgruppe P $2_1$/c (monoklin) mit a = 12,2189(9) Å, b = 13,6109(9) Å, c = 15,9826(11) Å, $\alpha$ = 90°, $\beta$ = 102,501(5)°, $\gamma$ = 90° und 4 Molekülen je Einheitszelle kristallisiert.

**Beispiel 1-2: Synthese des Kobalt-Komplexes [Co(babhq)OC(O)$CF_3$]**

Der Kobalt-Komplex [Co(babhq)OC(O)$CF_3$] wurde in zwei Stufen synthetisiert. In der ersten Stufe wurde gemäß Formel (XXXXV) der Komplex [Co(babhq)] hergestellt. Der Kobalt(II)-Komplex ist bekannt aus Wershofen, Stefan; Klein, Stephan; Jacob, Andreas; Sundermeyer, Joerg; Mei, Fuming Ger. Offen. (2009), DE 102008006881 A1 20090806 und Jacob, Andreas; Wershofen, Stefan; Klein, Stephan; Sundermeyer, Joerg; Mei, Fuming, PCT Int. Appl. (2009), WO 2009095164 A1 20090806.

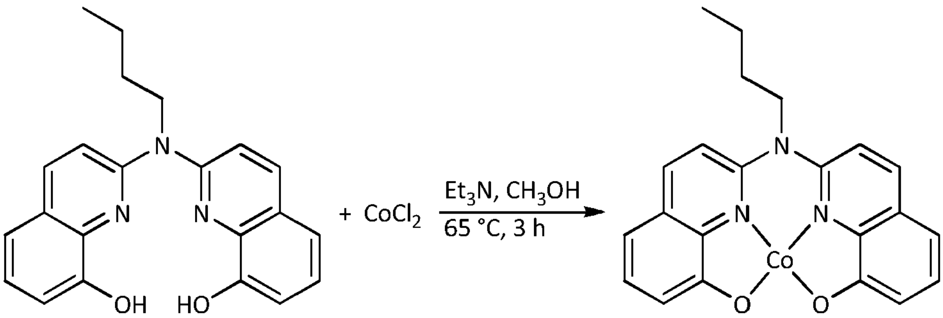


(XXXXV)

Der Ligand $H_2$(babhq) aus Beispiel 1 (1,000 mg, 2,78 mmol, 1,0 eq.) wurde in Methanol (45 mL) vorgelegt und die Mischung auf 50 °C erwärmt. Anschließend wurde eine Lösung von Kobaltchlorid-Hexahydrat (674 mg, 2,83 mmol, 1,0 eq.) in entgastem Wasser (5 mL) sowie Triethylamin (850 µL, 6,13 mmol, 2,2 eq.) zugegeben. Es trat sofort eine Farbveränderung nach rot-orange ein. Die Mischung wurde unter Rückfluss für drei Stunden erhitzt. Der ausgefallene rote Feststoff wurde abfiltriert, mit Ether (20 mL) gewaschen und unter Feinvakuum getrocknet Ausbeute: 870 mg (2,09 mmol, 75%), rotbrauner Feststoff.

HR-ESI/MS ($CH_3OH$): m/z berechnet für $[C_{22}H_{19}CoN_3O_2]^+$: 416,0804, gefunden: 416,0804.

CHN Analyse: $C_{22}H_{19}CoN_3O_2$ (416,34 g/mol) berechnet: C, 63,47; H, 4,60; N, 10,09 wt%; gefunden: C, 62,94;

H, 4,49; N, 9,94 wt%.

In der zweiten Stufe wurde gemäß Formel (XXXXVI) der Co(II)-Komplex mit Di(trifluoracetoxy)iodosobenzol oxidiert.

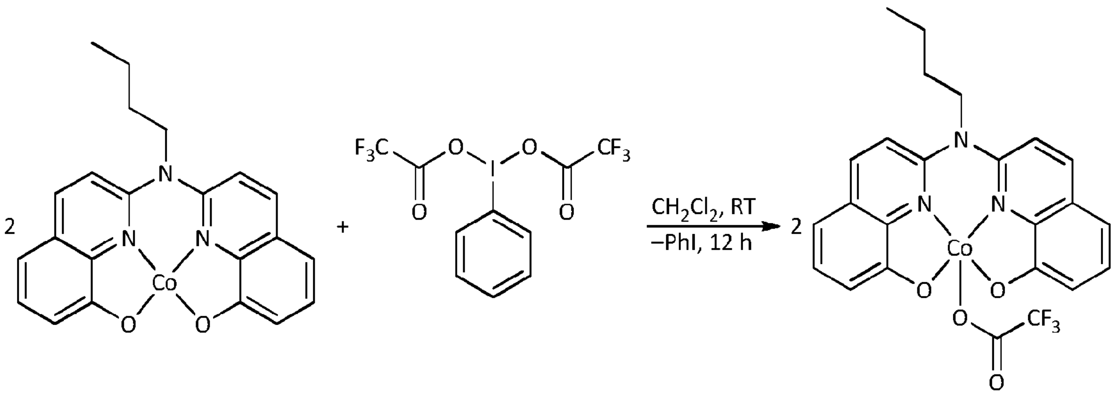


(XXXXVI)

[Co(babhq)] aus Stufe eins (722 mg, 1,73 mmol, 1,9 eq.) wurden in Methylenchlorid (40 mL) suspendiert und mit Di(trifluoracetoxy)iodosobenzol (392 mg, 0,91 mmol, 1,0 eq.) versetzt, wobei die Mischung sofort dunkler wurde. Diese wurde bei Raumtemperatur über Nacht gerührt. Nach Zugabe von Diethylether (10 mL) wurde der ausgefallene Feststoff abgetrennt, mit Diethylether (10 mL) gewaschen und unter Feinvakuum getrocknet.

Ausbeute: 750 mg (1,42 mmol, 81%), graubrauner Feststoff.

HR-ESI/MS ($CH_3OH$): m/z berechnet für $[C_{24}H_{19}CoF_3N_3O_4+Na]^+$: 552,0552, gefunden: 552,0559.

$^1H$-NMR (300,1 MHz, $(CD_3)_2SO$): $\delta$ (ppm) = 0,97 (t, $J$= 7,3 Hz, 3H, $CH_3$), 1,50 (td, $J$ = 14,9, 7,4, 2H, $CH_2CH_3$), 1,86-2,07 (m, 2H, $CH_2Et$), 4,52-4,80 (m, 2H, $NCH_2$), 7,27 (dd, $J$ = 7,2, 1,7 Hz, 2H, H5), 7,45-7,66 (m, 4H, H6/H7), 7,92 (d, $J$ = 9,4 Hz, 2H, H3), 8,63 (d, $J$ = 9.4 Hz, 2H, H4).

$^{13}C\{1H\}$-NMR (62,9 MHz, $(CD_3)_2SO$): $\delta$ (ppm) = 13,2 ($CH_3$), 18,4 ($CH_2CH_3$), 29,3 ($CH_2Et$), 34,0 ($CF_3$), 49,9 ($NCH_2$), 111,8 (C5), 114,2 (C3), 115,8 (C7), 125,2 (C4a), 127,9 (C6), 140,2 (C4), 145,5 (C8a), 149,4 (C2), 166,6 (C8). Der Kohlenstoff der $CF_3$-Gruppe konnte nicht aufgelöst werden.

CHN Analyse: $C_{25}H_{20}CoF_3N_2O_4 \cdot 1/4\ CH_2Cl_2$ (549,90 g/mol) berechnet: C, 52,90; H, 3,57; N, 7,63 wt%; gefunden: C, 52,74; H, 3,54; N, 7,66 wt%.

**Beispiel 1-3: Synthese des Zink-Komplexes [Zn(babhq)]**

Der Komplex [Zn(babhq)] (CAS 252565-55-2) ist bekannt aus Heuer, Helmut-Werner; Wehrmann, Rolf; Elschner Andreas; (Bayer AG), DE 19981025737 A1 (1999) und wurde gemäß Formel (XXXXVII) hergestellt.

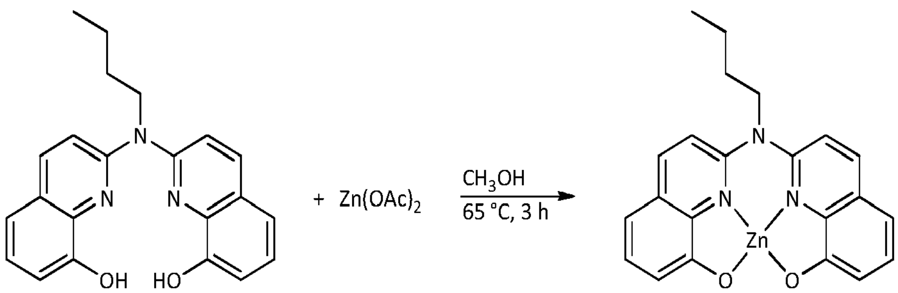


(XXXXVII)

Der Ligand $H_2$(babhq) (715 mg, 2,0 mmol, 1,0 eq.) aus Beispiel 1 wurde in 60 mL Methanol gelöst und mit Zinkacetat-dihydrat (437 mg, 2,0 mmol, 1,0 eq.) versetzt. Die Mischung wurde erhitzt, wobei ein gelblicher Feststoff ausfiel. Nach 3 h Rühren bei Siedetemperatur wurde abgekühlt und der ausgefallene Feststoff abgetrennt. Dieser wurde mit 15 mL Ether gewaschen und unter Feinvakuum getrocknet.

Ausbeute: 615 mg (1,5 mmol, 73%), hellgelbes Pulver.

HR-ESI/MS ($CH_3OH$): m/z berechnet für $[C_{22}H_{20}N_3O_2Zn]^+$: 422,0842, gefunden: 422,0832.

$^1H$-NMR (300,1 MHz, $(CD_3)_2SO$): $\delta$ (ppm) = 0,79 (t, $J$ = 7,3 Hz, 3H, $CH_3$), 1,31 (td, $J$ = 14,5, 7,4 Hz, 2H, $CH_2CH_3$), 1,61-1,74 (m, 2H, CH2Et), 4,38 (t, $J$ = 6,7 Hz, 2H, NCH2), 6,79 (dd, $J$ = 7,8, 0,9 Hz, 2H, H7), 6,88 (dd, $J$ = 7,8, 0,9 Hz, 2H, H5), 7,28 (t, $J$ = 7,9 Hz, 2H, H6), 7,66 (d, $J$ = 9,1 Hz, 2H, H3), 8,36 (d, $J$ = 9,1 Hz, 2H, H4).

$^{13}C\{^1H\}$-NMR (75,5 MHz, $(CD_3)_2SO$): δ (ppm) = 13,3 ($CH_3$), 18,7 ($CH_2CH_3$), 30,2 ($CH_2Et$), 49,7 ($NCH_2$), 108,6 (C5), 112,4 (C7), 114,6 (C3), 125,1 (C4a), 127,9 (C6), 136,9 (C8a), 140,0 (C4), 151,8 (C2), 161,3 (C8).

CHN Analyse: $C_{22}H_{19}N_3O_2Zn \cdot 1/2\ H_2O$ (431,82 g/mol) berechnet: C, 61,19; H, 4,67; N, 9,73 wt%; gefunden: C, 61,14; H, 4,61; N, 9,69 wt%.

Einkristall-Röntgenanalyse: Kristalle der Zusammensetzung $[Zn(babhq)CH_3OH] \cdot 2CH_3OH$ wurden durch Umkristallisieren der Verbindung aus Methanol/Ether bei Raumtemperatur als hellgelbe Plättchen erhalten. Eine Einkristall-Röntgenanalyse eines $0{,}40 \cdot \times 0{,}14 \cdot \times 0{,}08\ mm^3$ großen Kristalls ergab, dass die Verbindung in der Raumgruppe P $2_1/n$ (monoklin) mit a = 13,5366(8) Å, b = 9,3828(14) Å, c = 19,4198(14) Å, $\alpha = 90°$, $\beta = 93{,}974(5)°$, $\gamma = 90°$ und 4 Molekülen je Einheitszelle kristallisiert.

**Beispiel 2-1: Synthese des Chrom-Komplexes $[Cr(bpphen)OC(O)CF_3]$**

Der Chrom-Komplex $[Cr(bpphen)OC(O)CF_3]$ wurde in zwei Stufen synthetisiert. In der ersten Stufe wurde gemäß Formel (XXXXVIII) der Komplex [Cr(bpphen)Cl] hergestellt.

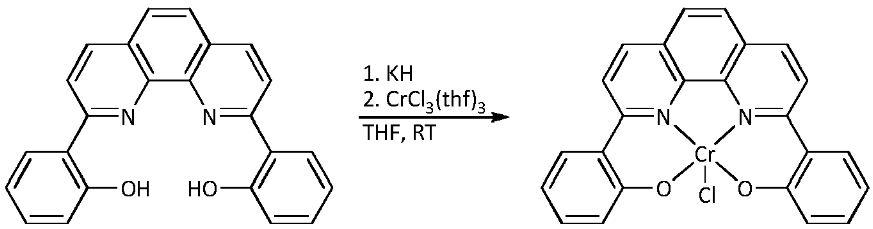


(XXXXVIII)

$H_2$(bpphen) aus Beispiel 2 (500 mg, 1,37 mmol, 1,0 eq.) und Kaliumhydrid (110 mg, 2,74 mmol, 2,0 eq.) wurden in trockenem THF (20 mL) suspendiert. Diese Mischung wurde gerührt bis die Gasentwicklung aufhörte. Eine Suspension von purpurfarbenen $[CrCl_3(thf)_3]$ (515 mg, 1,37 mmol, 1,0 eq.) in trockenem THF (20 mL) wurde hinzugefügt, wobei sich die Reaktionsmischung braun verfärbte. Nach 12-stündigem Rühren bei Raumtemperatur wurde Wasser (100 mL) hinzugefügt. Der gebildete Niederschlag wurde abfiltriert, mit Wasser gewaschen und unter Feinvakuum bei 50 °C getrocknet.

Ausbeute: 540 mg (1,20 mmol, 88%), brauner Feststoff.

HR-ESI/MS ($CH_3OH$): m/z berechnet für $[C_{24}H_{14}CrN_2O_2]^+$: 414,0455, gefunden: 414,0452.

CHN Analyse: berechnet für $C_{24}H_{14}CrN_2O_2 \bullet 3\ H_2O$ (503,88 g/mol): C, 57,21; H, 4,00; N, 5,56 wt%; gefunden: C, 57,47; H, 3,79; N, 5,11 wt%.

In der zweiten Stufe wurde gemäß Formel (IL) der Komplex [Cr(bpphen)Cl] mit Silbertrifluoracetat umgesetzt.

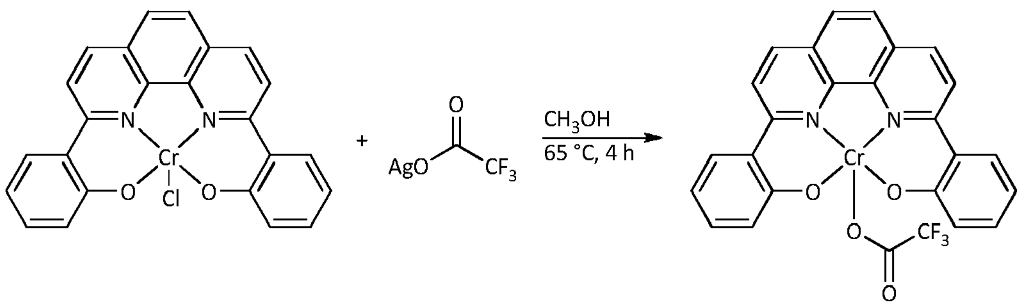


(IL)

[Cr(bpphen)Cl] aus Stufe eins (128 mg, 0,29 mmol, 1,0 eq.) wurde in Ethanol (15 ml) suspendiert. Unter Rühren wurde Silbertrifluoracetat (63 mg, 0,29 mmol, 1,0 eq.) hinzugefügt. Die Mischung wurde unter Rückfluss für 4 h gekocht. Nach Abkühlen auf Raumtemperatur wurden die festen Bestandteile durch Zentrifugieren abgetrennt. Sie wurden mehrmals mit heißem Ethanol extrahiert. Die vereinigten Lösungen wurden zur Trockene eingedampft und unter Feinvakuum bei 50 °C getrocknet.

Ausbeute: 118 mg (0,22 mmol, 79%), brauner Feststoff

HR-ESI/MS ($CH_3CN$): m/z berechnet für $[C_{26}H_{14}CrF_3N_2O_4+Na]^+$: 550,0204, gefunden: 550,0203.

CHN Analyse: $C_{26}H_{14}CrF_3N_2O_4$ (527,39 g/mol) berechnet: C, 59,21; H, 2,98; N, 5,31 wt%; gefunden: C, 49,67; H, 2,78; N, 5,18 wt%.

Einkristall-Röntgenanalyse: Kristalle der Zusammensetzung $[Cr(bpphen)OC(O)CF_3]_2$ wurden durch Umkristal-

lisieren der Verbindung aus DMF/Ether bei Raumtemperatur als rote Prismen erhalten. Eine Einkristall-Röntgenanalyse eines 0,30·× 0,18·× 0,15 $mm^3$ großen Kristalls ergab, dass die Verbindung in der Raumgruppe P $2_1/a$ (monoklin) mit a = 11,2256(4) Å, b = 24,1915(6) Å, c = 11,4141(4) Å, $\alpha$ = 90°, $\beta$ = 106,099(3)°, $\gamma$ = 90° und 4 Molekülen je Einheitszelle kristallisiert.

**Beispiel 2-2: Synthese des Kobalt-Komplexes [Co(bpphen)OC(O)$CF_3$]**

Der Kobalt-Komplex [Co(bpphen)OC(O)$CF_3$] wurde in zwei Stufen synthetisiert. In der ersten Stufe wurde gemäß Formel (L) der Komplex [Co(bpphen)] hergestellt. Der Kobalt(II)-Komplex (CAS 208171-82-8) ist bekannt aus Wershofen, Stefan; Klein, Stephan; Jacob, Andreas; Sundermeyer, Joerg; Mei, Fuming Ger. Offen. (2009), DE 102008006881 A1 20090806 und Jacob, Andreas; Wershofen, Stefan; Klein, Stephan; Sundermeyer, Joerg; Mei, Fuming, PCT Int. Appl. (2009), WO 2009095164 A1 20090806 und Orejon, Aranzazu; Castellanos, Aida; Salagre, Pilar; Castillon, Sergio; Claver, Carmen, Can. J. Chem. 2005, 83, 764-768 wie auch Routier, Sylvain; Joanny, Valerie; Zaparucha, Anne; Vezin, Herve; Catteau, Jean-Pierre; Bernier, Jean-Luc; Bailly, Christian, J. Chem. Soc., Perkin Trans. 2 1998, 863-868.

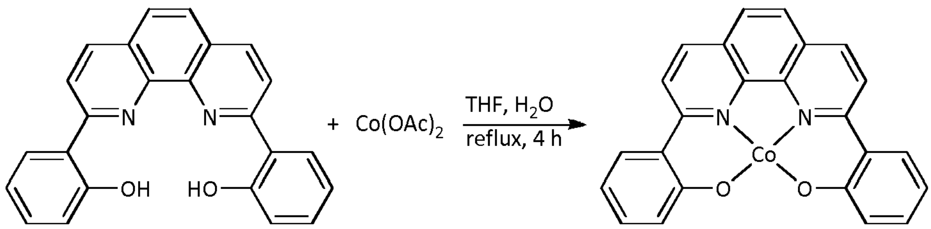


(L)

Der Ligand $H_2$(bpphen) aus Beispiel 2 (984 mg, 2,70 mmol, 1,0 eq.) wurde in Tetrahydrofuran (40 mL) vorgelegt und die Mischung auf 50 °C erwärmt. Anschließend wurde eine Lösung von Kobalt(II)acetat-tetrahydrat (673 mg, 2,70 mmol, 1,0 eq.) in entgastem Wasser (10 mL) zugegeben. Es trat sofort eine Farbveränderung nach rot ein. Die Mischung wurde unter Rückfluss für vier Stunden erhitzt. Der ausgefallene rote Feststoff wurde abfiltriert, mit Ether (20 mL) gewaschen und unter Feinvakuum getrocknet

Ausbeute: 946 mg (2,25 mmol, 83%), roter Feststoff.

HR-ESI/MS ($CH_3OH$): m/z berechnet für $[C_{24}H_{14}CoN_2O_2]^+$: 421,0382, gefunden: 421,0385.

CHN Analyse: $C_{24}H_{14}CoN_2O_2$ (421,31 g/mol) berechnet: C, 68,42; H, 3,35; N, 6,65 wt%; gefunden: C, 67,86; H, 3,37; N, 6,55 wt%.

In der zweiten Stufe wurde gemäß Formel (LI) der Co(II)-Komplex mit Di(trifluoracetoxy)iodosobenzol oxidiert.

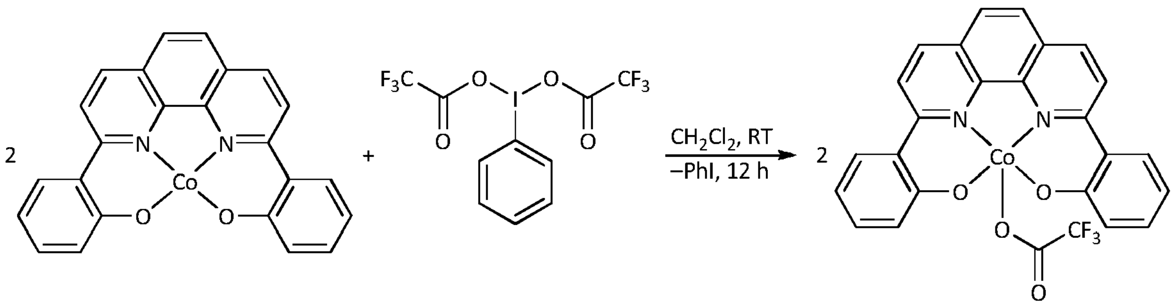


(LI)

[Co(bpphen)] aus Stufe eins (506 mg, 1,20 mmol, 1,9 eq.) wurden in Tetrahydrofuran (20 mL) suspendiert und mit Di(trifluoracetoxy)iodosobenzol (268 mg, 0,62 mmol, 1,0 eq.) versetzt, wobei die Mischung sofort dunkler wurde. Diese wurde bei Raumtemperatur über Nacht gerührt. Nach Zugabe von Diethylether (10 mL) wurde der ausgefallene Feststoff abgetrennt, mit Diethylether (10 mL) gewaschen und unter Feinvakuum getrocknet.

Ausbeute: 550 mg (1,03 mmol, 86%), brauner Feststoff.

HR-ESI/MS ($CH_3OH$): m/z berechnet für $[C_{26}H_{14}CoF_3N_2O_4+Na]^+$: 557,0130, gefunden: 557,0147.

$^1$H-NMR (300,1 MHz, $(CD_3)_2SO$): $\delta$ (ppm) = 6,88 (t, *J* = 7,3 Hz, 2H, H4), 7,47 (t, *J* = 7,5 Hz, 2H, H3), 7,87 (d, *J* = 7,9 Hz, 2H, H2), 8,37 (s, 2H, H12), 8,41 (m, *J* = 7,4 Hz, 2H, H5), 8,93 (d, *J* = 8,9 Hz, 2H, H9), 9,01 (d, *J* = 8,9 Hz, 2H, H10).

$^{13}$C{$^1$H}-NMR (62,9 MHz, $(CD_3)_2SO$): $\delta$ (ppm) = 116,3 (C4), 117,2 (C6), 121,9 (C9), 124,6 (C2), 125,9 (C12),

127,5 (C11), 129,2 (C5), 132,9 (C3), 138,6 (C10), 147,7 (C13), 155,3 (C7), 165,1 (C1). Der Kohlenstoff der $CF_3$-Gruppe konnte nicht aufgelöst werden.

CHN Analyse: $C_{25}H_{14}CoF_3N_2O_4$ (534,33 g/mol) berechnet: C, 58,44; H, 2,64; N, 5,24 wt%; gefunden: C, 58,01; H, 2,71; N, 5,14 wt%.

**Beispiel 2-3: Synthese des Zink-Komplexes [Zn(bpphen)]**

Der Ligand $H_2$(bpphen) (700 mg, 1,9 mmol, 1,0 eq.) aus Beispiel 2 wurde in 40 mL Methanol gelöst und gemäß Formel (LII) mit Zinkacetat-dihydrat (422 mg, 1,9 mmol, 1,0 eq.) versetzt. Die Mischung wurde erhitzt, wobei ein gelblicher Feststoff ausfiel. Nach 3 h Rühren bei Siedetemperatur wurde abgekühlt und der ausgefallene Feststoff abgetrennt. Dieser wurde mit 10 mL Ether gewaschen und unter Feinvakuum getrocknet.

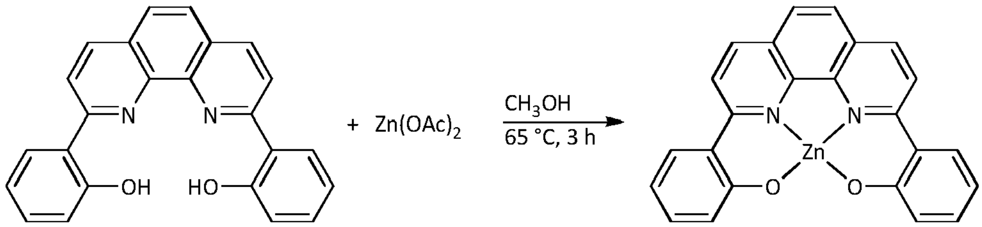


(LII)

Ausbeute: 661 mg (1,6 mmol, 80%), gelbes Pulver.

HR-APCI/MS ($CH_3OH$): m/z berechnet für $[C_{24}H_{14}N_2O_2Zn+H]^+$: 427,0420, gefunden: 427,0420.

$^1$H-NMR (300,1 MHz, $(CD_3)_2SO$): δ (ppm) = 6,57 (ddd, *J* = 8,1, 6,9, 1,3 Hz, 2H, H4), 6,83 (dd, *J* = 8,4, 1,2 Hz, 2H, H2), 7,22 (ddd, *J* = 8,5, 6,9, 1,7 Hz, 2H, H3), 7,93 (dd, *J* = 8,2, 1,7 Hz, 2H, H5), 8,03 (s, 2H, H12), 8,42 (d, *J* = 9,1 Hz, 2H, H9), 8,65 (d, *J* = 8,9 Hz, 2H, H10).

$^{13}C\{^1H\}$-NMR (75,5 MHz, $(CD_3)_2SO$): 113,3 (C4), 119,7 (C6), 122,6 (C9), 124,3 (C2), 124,8 (C12), 125,4 (C11), 129,8 (C5), 132,3 (C3), 138,2 (C13), 138,5 (C10), 158,6 (C7), 170,2 (C1).

CHN Analyse: $C_{24}H_{14}N_2O_2Zn$ (427,79 g/mol) berechnet: C, 67,38; H, 3,30; N, 6,55 wt%; gefunden: C, 66,82; H, 3,40; N, 6,47 wt%.

**Beispiel 3-1: Synthese des Zink-Komplexes [Zn(nbhq)]**

Der Ligand $H_2$(nbhq) aus Beispiel 3 (630 mg, 1,52 mmol, 1,0 eq.) wurde in Methanol (60 mL) gelöst und gemäß Formel (LIII) Zinkacetat-dihydrat (334 mg, 1,52 mmol, 1,0 eq.) hinzugefügt. Diese Reaktionsmischung wurde zwei Stunden unter Rückfluss gekocht. Nach Abkühlen auf Raumtemperatur wurde ein Niederschlag durch Filtration abgetrennt, mit Ether (15 mL) gewaschen und im Feinvakuum getrocknet.

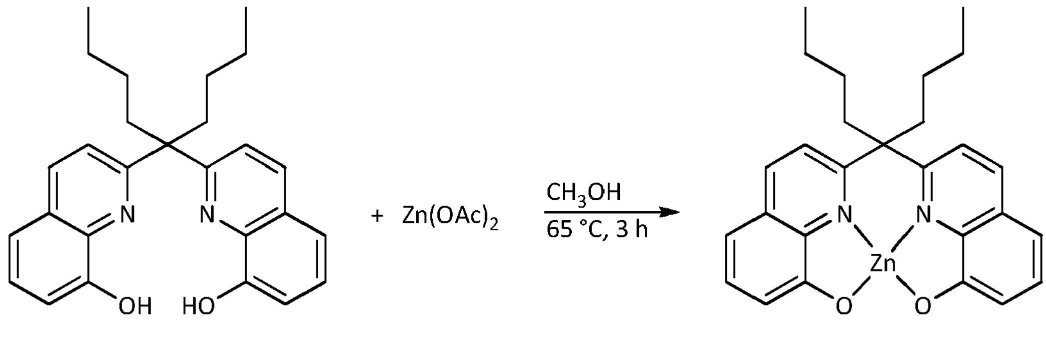


(LIII)

Ausbeute: 647 mg (1,35 mmol, 89%), hellgelbes Pulver

HR-ESI/MS ($CH_3OH$): m/z berechnet für $[C_{27}H_{28}N_2O_2Zn+Na]^+$: 499,1334, gefunden: 499,1344.

$^1$H-NMR (300,1 MHz, $(CD_3)_2SO$): δ (ppm) = 0,75 (t, J = 6,9 Hz, 6H, $CH_3$), 0,83-1,06 (m, 4H, $CH_2$Et), 1,23 (s, 4H, $CH_2CH_3$), 2,47 (s, 4H, $CCH_2$), 6,89 (d, J = 8,0 Hz, 2H, H7), 6,78 (d, J = 7,7 Hz, 2H, H5), 7,36 (t, J = 7,9 Hz, 2H, H6), 7,88 (d, J = 8,8 Hz, 2H, H3), 8,37 (d, J = 8,9 Hz, 2H, H4).

$^{13}C\{^1H\}$-NMR (75,5 MHz, $(CD_3)_2SO$): δ (ppm) = 13,7 ($CH_3$), 22,3 ($CH_2CH_3$), 26,6 ($CH_2$Et), 40,3 ($CCH_2$), 51,9 ($CCH_2$), 108,4 (C5), 111,9 (C7), 120,5 (C3), 127,3 (C4a), 129,8 (C6), 138,8 (C4), 139,0 (C8a), 159,9 (C2), 162,4 (C8).

CHN Analyse: berechnet für $C_{27}H_{28}N_2O_2Zn$ (477,93 g/mol): C, 67,85; H, 5,91; N, 5,86 wt%; gefunden: C, 59,31;

H, 5,83; N, 4,65 wt%.

**Beispiel 1-1-1: Synthese des Polycyclohexencarbonats mit dem Komplex [Cr(babhq)OC(O)$CF_3$] aus Beispiel 1-1 in Gegenwart von PPNCl**

Polycyclohexencarbonat wurde gemäß der Formel (LIV) durch Umsetzung von Cyclohexenoxid mit $CO_2$ hergestellt. Die in der Formel (LIV) angegebenen Buchstaben bezeichnen dabei die Kennzeichnung der Atome für die Zuordnung der Signale in den NMR-Spektren.

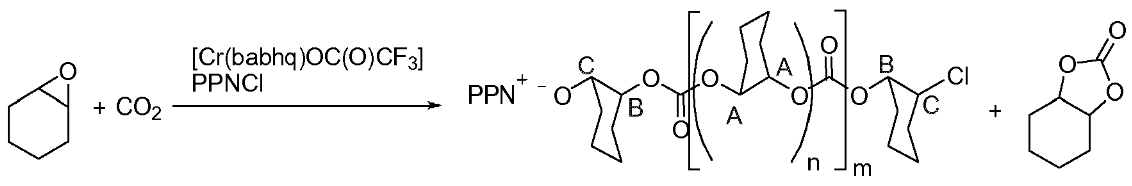


(LIV)

In einem 160 ml Autoklaven, ausgestattet mit einem Hohlwellenrührer, wurden Cr(III) Katalysator [Cr(babhq) OC(O)$CF_3$] (13,6 mg, 0,026 mmol), Bis(triphenylphosphoranylidene)ammonium chlorid (PPNCl, 34,8 mg, 0.061 mmol) und Cyclohexenoxid (15,3 ml, 151 mmol) vorgelegt und bei Raumtemperatur mit $CO_2$ (20 bar) beaufschlagt (molares Verhältnis Cyclohexenoxid/Katalysator/PPNCl = 4885/1/2,3). Anschließend wurde die Reaktionsmischung auf 100 °C aufgeheizt (innerhalb von 10 min) und mit 700 rpm für 10 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Das über in-situ IR Spektroskopie gemessene Zeit-Konzentrationsprofil ist in Fig. 1 gezeigt.

Menge des nicht umgesetzten Cyclohexenoxids: 24,0 %

Ausbeute an cyclischem Cyclohexencarbonat: 0,8%

Ausbeute an linearem Cyclohexencarbonat: 75,2 %

Als Rückstand verblieb ein hochviskoses Material, das in Methylenchlorid gelöst und in einen 250 ml Rundkolben überführt wurde. Nach Abdestillieren der flüchtigen Bestandteile am Rotationsverdampfer im Wasserbad (50 °C) wurde das Polymer als hell-oranges Pulver erhalten.

Eine Probe des Pulvers wurde in $CDCl_3$ gelöst und über $^1H$ NMR Spektroskopie untersucht.

Es wurden nur die charakteristischen Signale des linearen Polycyclohexencarbonats gebildet durch alternierenden Einbau von Cyclohexenoxid und Kohlendioxid in die Polymerkette beobachtet.

$^1H$-NMR (pCHC, $CDCl_3$) $\delta$ (ppm) = 4,51 (m, $H^A$), 4,28 (m, $H^B$), 3,41 (m, $H^C$).

Die Analyse des Produktes mit GPC ergab $M_n$ = 3831 g/mol und einen PDI von 1,20.

**Beispiel 1-1-2: Synthese des Polycyclohexencarbonats mit dem Komplex [Cr(babhq)OC(O)$CF_3$] aus Beispiel 1-1 in Gegenwart von TBACl und 1,8-Octandiol**

Polycyclohexencarbonat wurde gemäß der Formel (LV) durch Umsetzung von Cyclohexenoxid mit $CO_2$ hergestellt. Die in der Formel (LV) angegebenen Buchstaben bezeichnen dabei die Kennzeichnung der Atome für die Zuordnung der Signale in den NMR-Spektren.

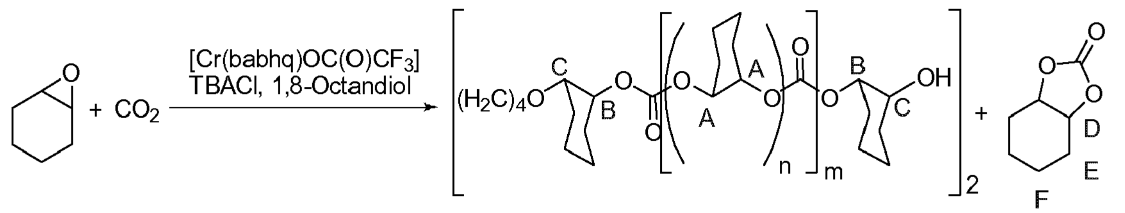


(LV)

In einem 160 ml Autoklaven, ausgestattet mit einem Hohlwellenrührer, wurden Cr(III) Katalysator [Cr(babhq) OC(O)$CF_3$] (12,3 mg, 0,022 mmol), Tetrabutylammonium chlorid (TBACl, 26,0 mg, 0.094 mmol), 1,8-Octandiol (121 mg, 0,828 mmol) und Cyclohexenoxid (8,6 ml, 85 mmol) vorgelegt und bei Raumtemperatur mit $CO_2$ (20 bar) beaufschlagt (molares Verhältnis Cyclohexenoxid/Katalysator/TBACl = 3542/1/3,9). Anschließend wurde die Reaktionsmischung auf 100 °C aufgeheizt (innerhalb von 10 min) und mit 700 rpm für 6 Stunden gerührt. Der Autoklav wurde dann mit Hilfe

eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Das über in-situ IR Spektroskopie gemessene Zeit-Konzentrationsprofil ist in Fig. 1 gezeigt.

Menge des nicht umgesetzten Cyclohexenoxids: 10,9 %

Ausbeute an cyclischem Cyclohexencarbonat: 20,1%

Ausbeute an linearem Cyclohexencarbonat: 69,0 %

Als Rückstand verblieb ein hochviskoses Material, das in Methylenchlorid gelöst und in einen 250 ml Rundkolben überführt wurde. Nach Abdestillieren der flüchtigen Bestandteile am Rotationsverdampfer im Wasserbad (50 °C) wurde das Polymer als hell-oranges Pulver erhalten.

Eine Probe des Pulvers wurde in $CDCl_3$ gelöst und über $^1H$ NMR Spektroskopie untersucht.

Es wurden die charakteristischen Signale des linearen Polycyclohexencarbonats gebildet durch nicht-alternierenden Einbau von Cyclohexenoxid und Kohlendioxid in die Polymerkette, Signale für in die Polymerkette eingebautes 1,8-Octandiol sowie die charakteristischen Signale des cyclischen Cyclohexencarbonats beobachtet.

$^1H$-NMR ($CDCl_3$) $\delta$ (ppm) = 4,49 (b, $H^A$, lineare Polycyclohexencarbonat-Einheiten), 4,34 (b, $H^A$, lineare Polycyclohexenether-Einheiten), 4,11 (b, $H^B$), 3,84-3,81 (m, $H^D$), 3,24 (b, $H^C$), 1,95 (m, $H^E$), 1,86-1,09 (m, $H^F$ und $CH_2$ von 1,8-Octandiol).

**Beispiel 1-1-3: Synthese des cyclischen Propylencarbonats mit dem Komplex [Cr(babhq)OC(O)$CF_3$] aus Beispiel 1-1 in Gegenwart von PPNCl**

Cyclisches Propylencarbonat wurde gemäß der Formel (LVI) durch Umsetzung von Propylenoxid mit $CO_2$ hergestellt. Die in der Formel (LVI) angegebenen Buchstaben bezeichnen dabei die Kennzeichnung der Atome für die Zuordnung der Signale in den NMR-Spektren.

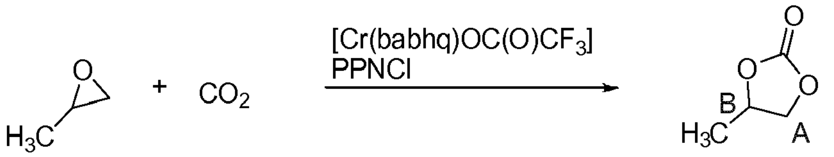


(LVI)

In einem 160 ml Autoklaven, ausgestattet mit einem Hohlwellenrührer, wurden Cr(III) Katalysator [Cr(babhq) OC(O)$CF_3$] (11 mg, 0,021 mmol) Bis(triphenylphosphoranylidene)ammonium chlorid (PPNCl, 29,0 mg, 0,050 mmol) und Propylenoxid (12,0 ml, 171 mmol) vorgelegt und bei Raumtemperatur mit $CO_2$ (20 bar) beaufschlagt (molares Verhältnis Propylenoxid/Katalysator/PPNCl = 8143/1/2,4). Anschließend wurde die Reaktionsmischung auf 100 °C aufgeheizt (innerhalb von 10 min) und mit 700 rpm für 3,5 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Propylenoxids: 13,0 %

Ausbeute an cyclischem Propylencarbonat: 87,0 %

Im Rückstand verblieb eine durch den Katalysator gelb-orange gefärbte Lösung, die in einen Rundkolben überführt wurde. Nach Abdestillieren der flüchtigen Bestandteile am Rotationsverdampfer im Wasserbad (50 °C) wurde das cyclische Propylencarbonat als gelborange Flüssigkeit erhalten.

Eine Probe wurde in $CDCl_3$ gelöst und über $^1H$ NMR Spektroskopie untersucht.

Es wurden nur die charakteristischen Signale des cyclischen Propylencarbonats beobachtet.

$^1H$-NMR (400 MHz, $CDCl_3$) $\delta$ (ppm) = 4,83-4,90 (1H, m, $H^B$), 4,57 (1H, dd, $H^A$), 4,04 (dd, $H^A$), 1,49 (3H, d, $CH_3$).

**Beispiel 1-1-4: Synthese des cyclischen Propylencarbonats mit dem Komplex [Cr(babhq)OC(O)$CF_3$] aus Beispiel 1-1 in Gegenwart von TBABr und 1,8-Octandiol**

Cyclisches Propylencarbonat wurde gemäß der Formel (LVII) durch Umsetzung von Propylenoxid mit $CO_2$ hergestellt. Die in der Formel (LVII) angegebenen Buchstaben bezeichnen dabei die Kennzeichnung der Atome für die Zuordnung der Signale in den NMR-Spektren.

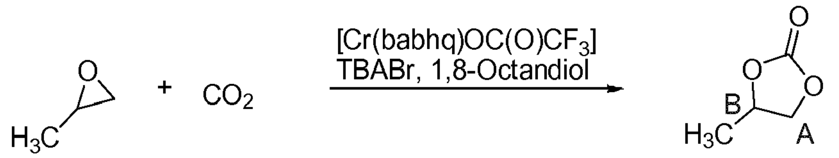


(LVII)

In einem 160 ml Autoklaven, ausgestattet mit einem Hohlwellenrührer, wurden Cr(III) Katalysator [Cr(babhq) OC(O)$CF_3$] (11,8 mg, 0,023 mmol), Tetrabutylammonium bromid (TBABr, 24,8 mg, 0,077 mmol), 1,8-Octandiol (126 mg, 0,862 mmol) und Propylenoxid (7,0 ml, 99 mmol) vorgelegt und bei Raumtemperatur mit $CO_2$ (20 bar) beaufschlagt (molares Verhältnis Propylenoxid/Katalysator/TBABr = 4304/1/3,3). Anschließend wurde die Reaktionsmischung auf 60 °C aufgeheizt (innerhalb von 5 min) und mit 700 rpm für 4 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Propylenoxids: 38,0 %

Ausbeute an cyclischem Propylencarbonat: 62,0 %

Im Rückstand verblieb eine durch den Katalysator gelb-orange gefärbte Lösung, die in einen Rundkolben überführt wurde. Nach Abdestillieren der flüchtigen Bestandteile am Rotationsverdampfer im Wasserbad (50 °C) wurde das cyclische Propylencarbonat als gelborange Flüssigkeit erhalten.

Eine Probe wurde in $CDCl_3$ gelöst und über $^1H$ NMR Spektroskopie untersucht.

Es wurden nur die charakteristischen Signale des cyclischen Propylencarbonats beobachtet.

$^1H$-NMR (400 MHz, $CDCl_3$) $\delta$ (ppm) = 4,83-4,90 (1H, m, $H^B$), 4,57 (1H, dd, $H^A$), 4,04 (dd, $H^A$), 1,49 (3H, d, $CH_3$).

**Beispiel 1-1-5: Synthese des cyclischen Propylencarbonats mit dem Komplex [Cr(babhq)OC(O)$CF_3$] aus Beispiel 1-1 in Gegenwart von TBACl und 1,8-Octandiol**

Cyclisches Propylencarbonat wurde gemäß der Formel (LVIII) durch Umsetzung von Propylenoxid mit $CO_2$ hergestellt. Die in der Formel (LVIII) angegebenen Buchstaben bezeichnen dabei die Kennzeichnung der Atome für die Zuordnung der Signale in den NMR-Spektren.

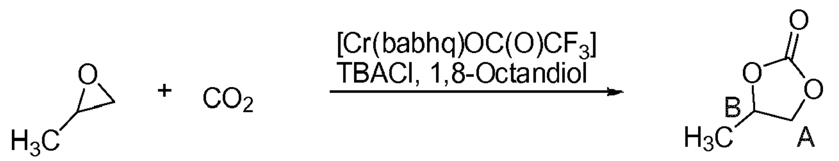


(LVIII)

In einem 160 ml Autoklaven, ausgestattet mit einem Hohlwellenrührer, wurden Cr(III) Katalysator [Cr(babhq) OC(O)$CF_3$] (10,6 mg, 0,020 mmol), Tetrabutylammonium chlorid (TBACl, 24,0 mg, 0,086 mmol), 1,8-Octandiol (126 mg, 0,862 mmol) und Propylenoxid (9,4 ml, 133 mmol) vorgelegt und bei Raumtemperatur mit $CO_2$ (20 bar) beaufschlagt (molares Verhältnis Propylenoxid/Katalysator/TBACl = 4304/1/4,1). Anschließend wurde die Reaktionsmischung auf 100 °C aufgeheizt (innerhalb von 10 min) und mit 700 rpm für 16 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Propylenoxids: < 1 %

Ausbeute an cyclischem Propylencarbonat: > 99 %

Im Rückstand verblieb eine durch den Katalysator gelb-orange gefärbte Lösung, die in einen Rundkolben überführt wurde. Nach Abdestillieren der flüchtigen Bestandteile am Rotationsverdampfer im Wasserbad (50 °C) wurde das cyclische Propylencarbonat als gelborange Flüssigkeit erhalten.

Eine Probe wurde in $CDCl_3$ gelöst und über $^1H$ NMR Spektroskopie untersucht.

Es wurden nur die charakteristischen Signale des cyclischen Propylencarbonats beobachtet.

$^1H$-NMR (400 MHz, $CDCl_3$) $\delta$ (ppm) = 4,83-4,90 (1H, m, $H^B$), 4,57 (1H, dd, $H^A$), 4,04 (dd, $H^A$), 1,49 (3H, d, $CH_3$).

**Beispiel 1-2-1: Synthese des cyclischen Propylencarbonats mit dem Komplex [Co(babhq)OC(O)$CF_3$] aus Beispiel 1-2 in Gegenwart von TBABr**

Cyclisches Propylencarbonat wurde gemäß der Formel (LIX) durch Umsetzung von Propylenoxid mit $CO_2$ hergestellt. Die in der Formel (LIX) angegebenen Buchstaben bezeichnen dabei die Kennzeichnung der Atome für die

Zuordnung der Signale in den NMR-Spektren.

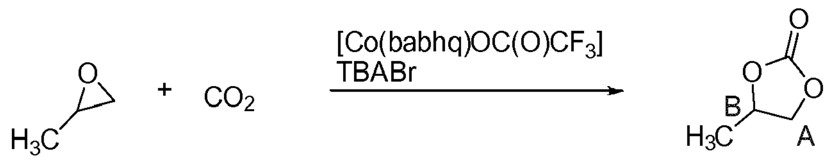


(LIX)

In einem 160 ml Autoklaven, ausgestattet mit einem Hohlwellenrührer, wurden Co(III) Katalysator [Co(babhq) OC(O)$CF_3$] (12,0 mg, 0,023 mmol), Tetrabutylammonium bromid (TBABr, 13,0 mg, 0,040 mmol) und Propylenoxid (7,2 ml, 102 mmol) vorgelegt und bei Raumtemperatur mit $CO_2$ (20 bar) beaufschlagt (molares Verhältnis Propylenoxid/ Katalysator/TBABr = 4435/1/1,7). Anschließend wurde die Reaktionsmischung auf 60 °C aufgeheizt (innerhalb von 5 min) und mit 700 rpm für 2,5 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Propylenoxids: 62,0 %

Ausbeute an cyclischem Propylencarbonat: 38,0 %

Im Rückstand verblieb eine durch den Katalysator gelb-orange gefärbte Lösung, die in einen Rundkolben überführt wurde. Nach Abdestillieren der flüchtigen Bestandteile am Rotationsverdampfer im Wasserbad (50 °C) wurde das cyclische Propylencarbonat als gelborange Flüssigkeit erhalten.

Eine Probe wurde in $CDCl_3$ gelöst und über $^1H$ NMR Spektroskopie untersucht.

Es wurden nur die charakteristischen Signale des cyclischen Propylencarbonats beobachtet.

$^1H$-NMR (400 MHz, $CDCl_3$) $\delta$ (ppm) = 4,83-4,90 (1H, m, $H^B$), 4,57 (1H, dd, $H^A$), 4,04 (dd, $H^A$), 1,49 (3H, d, $CH_3$).

**Beispiel 2-1-1: Synthese des cyclischen Propylencarbonats mit dem Komplex [Co(bpphen)OC(O)$CF_3$] aus Beispiel 1-2 in Gegenwart von PPNCl und 1-Octanol**

Cyclisches Propylencarbonat wurde gemäß der Formel (LX) durch Umsetzung von Propylenoxid mit $CO_2$ hergestellt. Die in der Formel (LX) angegebenen Buchstaben bezeichnen dabei die Kennzeichnung der Atome für die Zuordnung der Signale in den NMR-Spektren.

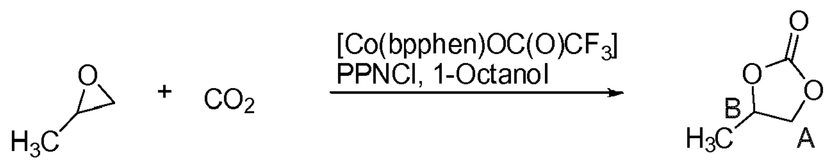


(LX)

In einem 160 ml Autoklaven, ausgestattet mit einem Hohlwellenrührer, wurden Co(III) Katalysator [Co(bpphen) OC(O)$CF_3$] (10,7 mg, 0,020 mmol), Bis(triphenylphosphoranylidene)ammonium chlorid (PPNCl, 23,0 mg, 0,040 mmol), 1-Octanol (0,31 ml, 2 mmol) und Propylenoxid (7,0 ml, 99 mmol) vorgelegt und bei Raumtemperatur mit $CO_2$ (20 bar) beaufschlagt (molares Verhältnis Propylenoxid/Katalysator/TBABr = 4950/1/2,0). Anschließend wurde die Reaktionsmischung auf 60 °C aufgeheizt (innerhalb von 5 min) und mit 700 rpm für 2,5 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Propylenoxids: 95,5 %

Ausbeute an cyclischem Propylencarbonat: 4,5 %

Im Rückstand verblieb eine durch den Katalysator gelb-orange gefärbte Lösung, die in einen Rundkolben überführt wurde. Nach Abdestillieren der flüchtigen Bestandteile am Rotationsverdampfer im Wasserbad (50 °C) wurde das cyclische Propylencarbonat als gelborange Flüssigkeit erhalten.

Eine Probe wurde in $CDCl_3$ gelöst und über $^1H$ NMR Spektroskopie untersucht.

Es wurden nur die charakteristischen Signale des cyclischen Propylencarbonats beobachtet.

$^1H$-NMR (400 MHz, $CDCl_3$) $\delta$ (ppm) = 4,83-4,90 (1H, m, $H^B$), 4,57 (1H, dd, $H^A$), 4,04 (dd, $H^A$), 1,49 (3H, d, $CH_3$).

**Beispiel 3-1-1: Synthese des cyclischen Cyclohexencarbonats mit dem Komplex [Zn(nbhq)] aus Beispiel 3-1 in Gegenwart von PPNCl und TBABr**

Cyclisches Cyclohexencarbonat wurde gemäß der Formel (LXI) durch Umsetzung von Cyclohexenoxid mit $CO_2$ hergestellt. Die in der Formel (LXI) angegebenen Buchstaben bezeichnen dabei die Kennzeichnung der Atome für die Zuordnung der Signale in den NMR-Spektren.

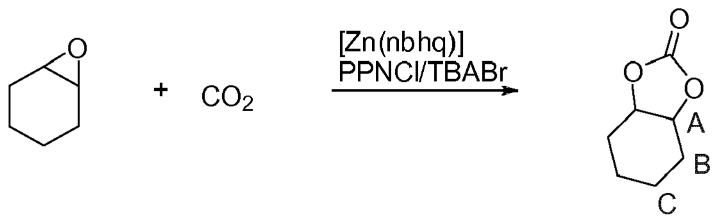


(LXI)

In einem 160 ml Autoklaven, ausgestattet mit einem Hohlwellenrührer, wurden Zn(II) Katalysator [Zn(nbhq)] (10,5 mg, 0,022 mmol), Bis(triphenylphosphoranylidene)ammonium chlorid (PPNCl, 30,0 mg, 0,052 mmol), Tetrabutylammoniumbromid (TBABr, 60 mg, 0,186 mmol) und Cyclohexenoxid (13,0 ml, 128 mmol) vorgelegt und bei Raumtemperatur mit $CO_2$ (20 bar) beaufschlagt (molares Verhältnis Cyclohexenoxid/Katalysator/(PPNCl+TBABr) = 6400/1/11,9). Anschließend wurde die Reaktionsmischung auf 100 °C aufgeheizt (innerhalb von 10 min) und mit 700 rpm für 3,5 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Cyclohexenoxids: 37,0 %

Ausbeute an cyclischem Cyclohexencarbonat: 63,0 %

Ausbeute an linearem Cyclohexencarbonat: 0 %

Als Rückstand verblieb eine durch den Katalysator gelb gefärbte Flüssigkeit, die in einen Rundkolben überführt wurde. Nach Abdestillieren der flüchtigen Bestandteile am Rotationsverdampfer im Wasserbad (50 °C) wurde das cyclische Cyclohexencarbonat als gelb-orange Lösung erhalten.

Eine Probe wurde in $CDCl_3$ gelöst und über $^1H$ NMR Spektroskopie untersucht.

Es wurden nur die charakteristischen Signale des cyclischen Cyclohexencarbonats beobachtet.

$^1H$-NMR(400 MHz, $CDCl_3$) $\delta$ (ppm) = 3,81 (2H, dd, $H^A$), 1,98 (4H, m, $H^B$), 0,97-1,01 (4H, m, $H^C$).

**Beispiel 3-1-2: Synthese des cyclischen Propylencarbonats mit dem Komplex [Zn(nbhq)] aus Beispiel 3-1 in Gegenwart von TBABr**

Cyclisches Propylencarbonat wurde gemäß der Formel (LXII) durch Umsetzung von Propylenoxid mit $CO_2$ hergestellt. Die in der Formel (LXII) angegebenen Buchstaben bezeichnen dabei die Kennzeichnung der Atome für die Zuordnung der Signale in den NMR-Spektren.

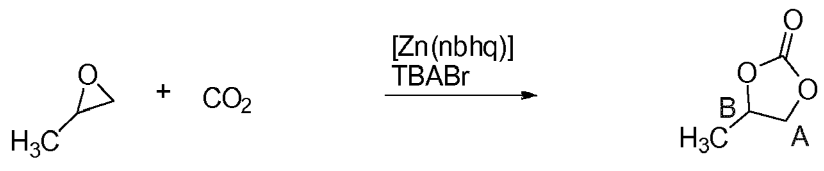


(LXII)

In einem 160 ml Autoklaven, ausgestattet mit einem Hohlwellenrührer, wurden Zn(II) Katalysator [Zn(nbhq)] (12,6 mg, 0,026 mmol), Tetrabutylammoniumbromid (TBABr, 26,0 mg, 0,081 mmol) und Propylenoxid (9,0 ml, 127 mmol) vorgelegt und bei Raumtemperatur mit $CO_2$ (20 bar) beaufschlagt (molares Verhältnis Propylenoxid/Katalysator/TBABr = 4885/1/3,1). Anschließend wurde die Reaktionsmischung auf 60 °C aufgeheizt (innerhalb von 5 min) und mit 700 rpm für 12 Stunden gerührt. Der Autoklav wurde dann mit Hilfe eines Eisbades auf 15 °C gekühlt und der verbleibende $CO_2$ Überdruck langsam abgelassen. Nach Öffnen des Autoklaven wurden eine NMR Probe für die quantitative Analyse entnommen und in $CDCl_3$ vermessen.

Menge des nicht umgesetzten Propylenoxids: 24,0 %

Ausbeute an cyclischem Propylencarbonat: 76,0 %

Ausbeute an linearem Propylencarbonat: 0 %

Als Rückstand verblieb eine durch den Katalysator gelb gefärbte Flüssigkeit, die in einen Rundkolben überführt wurde. Nach Abdestillieren der flüchtigen Bestandteile am Rotationsverdampfer im Wasserbad (50 °C) wurde das cyclische Propylencarbonat als gelb-orange Lösung erhalten.
Eine Probe wurde in $CDCl_3$ gelöst und über $^1H$ NMR Spektroskopie untersucht.
Es wurden nur die charakteristischen Signale des cyclischen Propylencarbonats beobachtet.
$^1H$-NMR (400 MHz, $CDCl_3$) $\delta$ (ppm) = 4,83-4,90 (1H, m, $H^B$), 4,57 (1H, dd, $H^A$), 4,04 (dd, $H^A$), 1,49 (3H, d, $CH_3$).

**Vergleich der Beispiele 1-1-1 bis 3-1-2**

Eine Übersicht über die erhaltenen Ergebnisse ist in der nachfolgenden Tabelle gezeigt.

Tabelle 1: Übersicht der Resultate der Beispiele 1-1-1 bis 3-1-2 (dabei bedeuten: PPNCl: Bis (triphenylphosphoranylidene)ammoniumchlorid; TBACl: Tetrabutylammoniumchlorid; TBABr: Tetrabutylammoniumbromid; 1,8-OL: 1,8-Octandiol; PO: Propylenoxid; CHO: Cyclohexenoxid)

| Beispiel | Als Katalysator eingesetzter Komplex | Co-Katalysator / Starter | Substrat | Nicht umgesetztes Epoxid | Ausbeute an cyclischem Carbonatester | Ausbeute an linearem Carbonatester |
|---|---|---|---|---|---|---|
| 1-1-1 | [Cr(babhq)OC(O)$CF_3$] | PPNCl / - | CHO | 24,0% | 0,8% | 75,2% |
| 1-1-2 | [Cr(babhq)OC(O)$CF_3$] | TBACl / 1,8-OL | CHO | 10,9% | 20,1% | 69,0% |
| 1-1-3 | [Cr(babhq)OC(O)$CF_3$] | PPNCl / - | PO | 13,0% | 87,0% | 0% |
| 1-1-4 | [Cr(babhq)OC(O)$CF_3$] | TBABr / 1,8-OL | PO | 38,0% | 62,0% | 0% |
| 1-1-5 | [Cr(babhq)OC(O)$CF_3$] | TBACl / 1,8-OL | PO | <1% | >99% | 0% |
| 1-2-1 | [Co(babhq)OC(O)$CF_3$] | TBABr / - | PO | 62,0% | 38,0% | 0% |
| 2-1-1 | [Co(bpphen)OC(O)$CF_3$] | PPNCl/ 1-Octanol | PO | 95,5 | 4,5 | 0% |
| 3-1-1 | [Zn(nbhq)] | PPNCl / TBABr | CHO | 37,0% | 63,0% | 0% |
| 3-1-2 | [Zn(nbhq)] | TBABr / - | PO | 24,0% | 76,0% | 0% |

Die Beispiele belegen, dass die als Katalysator eingesetzten Komplexe [Cr(babhq)OC(O)$CF_3$], [Co(babhq)OC(O)$CF_3$] und [Zn(nbhq)] für die Bildung von linearen und cyclischen Carbonatestern aus den als Epoxiden eingesetzten Verbindungen Cyclohexenoxid und Propylenoxid und Kohlendioxid aktiv sind.

**Figuren**

Figur 1 beschreibt das Zeit-Konzentrationsprofil für die in Beispiel 1-1-1 beschriebene Umsetzung von Cyclohexenoxid und Kohlendioxid mit [Cr(babhq)OC(O)$CF_3$]. Auf der x-Achse ist die Zeit in Minuten, auf der y-Achse der Molenbruch in mol/mol aufgetragen. Die abfallende Kurve beschreibt die Konzentration des Cyclohexenoxids, die aufsteigende Kurve die Konzentration des linearen Cyclohexencarbonats. Die Konzentration des cyclischen Cyclohexencarbonats lag unterhalb der Nachweisgrenze. Eine durch die Messpunkte größtenteils verdeckte durchgezogene Linie gibt eine mathematische Modellierung der gemessenen Daten gemäß einer Reaktion erster Ordnung wieder.
Figur 2 beschreibt das Zeit-Konzentrationsprofil für die in Beispiel 1-1-3 beschriebene Umsetzung von Propylenoxid und Kohlendioxid mit [Cr(babhq)OC(O)$CF_3$]. Auf der x-Achse ist die Zeit in Minuten, auf der y-Achse der Molenbruch in mol/mol aufgetragen. Die abfallende Kurve beschreibt die Konzentration des Propylenoxids, die aufsteigende Kurve die Konzentration des cyclischen Propylencarbonats. Die durchgezogene Linie gibt eine mathematische Modellierung der gemessenen Daten gemäß einer Reaktion zweiter Ordnung wieder.

## Patentansprüche

1. Verfahren zur Herstellung von linearen und/oder cyclischen Carbonatestern, umfassend den Schritt der Reaktion eines Epoxids mit Kohlendioxid in Gegenwart eines Katalysators,
**dadurch gekennzeichnet, dass**
der Katalysator einen Komplex eines Metalls M oder M-A mit einem Liganden L umfasst, wobei das Metall M in einer Oxidationsstufe von $\geq 0$ vorliegt,
A für Halogenid, Carboxylat, Phenolat, Sulfonat, Phosphonat, Alkyl, Alkoxy oder Amido steht und der Ligand L die nachfolgende Struktur (Ia) oder (Ib) aufweist, wobei weiterhin eine oder beide der in (Ia) und/oder (Ib) gezeigten OH-Gruppen deprotoniert sein können:

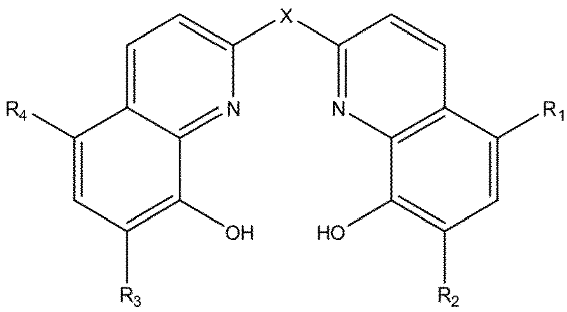

(Ia)

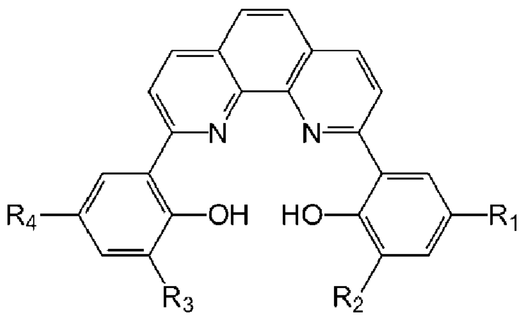

(Ib)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen und X für eine Brücke der Formel (II) oder (III) steht:

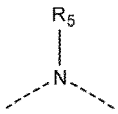

(II)

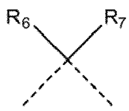

(III)

wobei R5 für einen $C_1$-$C_{22}$-Alkylrest, einen $C_5$-$C_{12}$-Cycloalkylrest, einen $C_7$-$C_{14}$-Aralkyl oder - Alkylarylrest, einen $C_6$-$C_{14}$-Arylrest, oder einen gegebenenfalls Alkyl-substituierten Pyridylrest oder Pyridylmethylrest steht und
R6 und R7 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest oder einen - COOR8-Rest stehen, wobei R8 für einen $C_1$-$C_8$-Alkylrest steht.

2. Verfahren gemäß Anspruch 1, wobei A für $OCOCH_3$, $OCOCF_3$, $OSO_2CF_3$, $OSO_2C_6H_5CH_3$, $OSO_2CF_3$, $OSO(CH_3)_2$ oder Halogenid steht.

3. Verfahren gemäß Anspruch 1, wobei M ausgewählt ist aus der Gruppe Zn(II), Cr(II), Mn(II), Mg(II), Fe(II), Co(II), Cr (III)-A, Mn(III)-A, Fe(III)-A und/oder Co(III)-A.

4. Verfahren gemäß Anspruch 1, wobei der Ligand L ausgewählt ist aus der Gruppe umfassend Verbindungen der Formeln (IV) bis (VII):

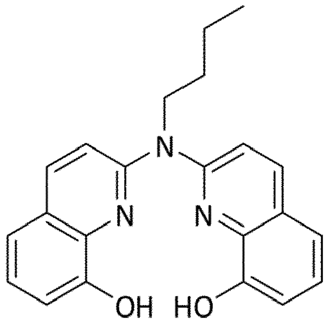

(IV)

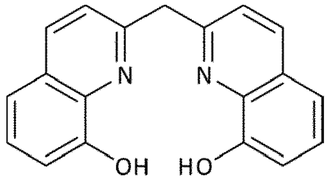

(V)

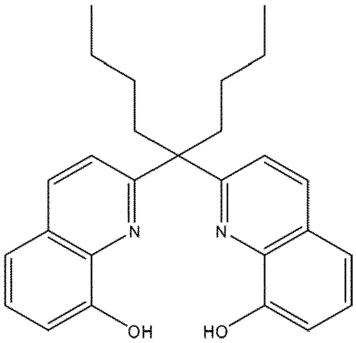

(VI)

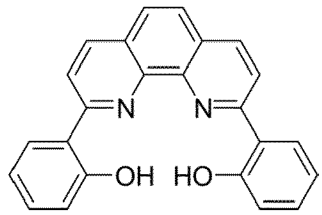

(VII)

5. Verfahren gemäß Anspruch 1, wobei der Katalysator ausgewählt ist aus der Gruppe umfassend Verbindungen der Formeln (VIII) bis (XVIII):

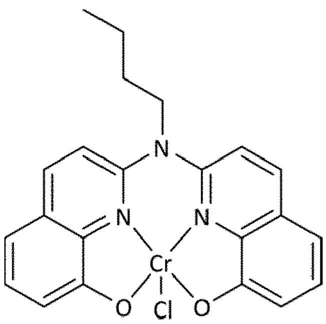

(VIII)

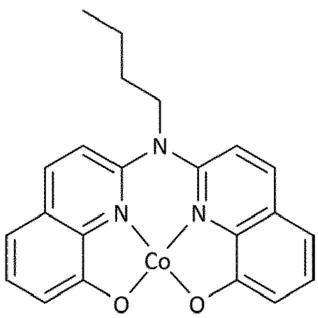

(IX)

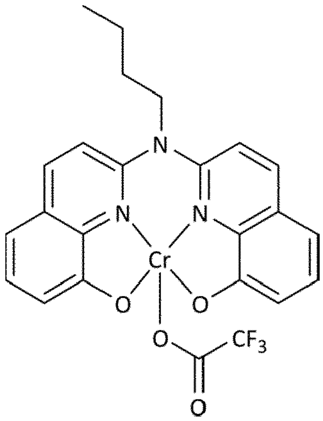

(X)

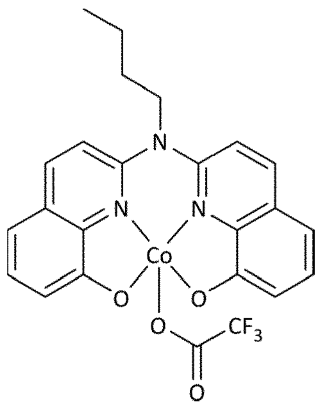

(XI)

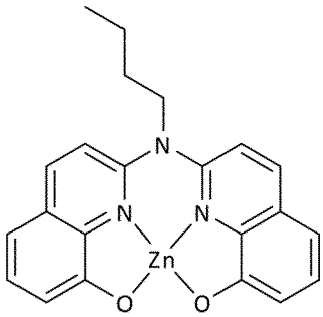

(XII)

(XIII)

(XIV)

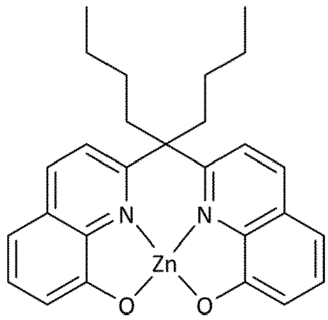

(XV)

(XVI)

(XVII)

(XVIII)

**6.** Verfahren gemäß Anspruch 1, wobei als Epoxid Ethylenoxid, Propylenoxid, Cyclohexenoxid oder Styroloxid oder beliebige Mischungen umfassend Ethylenoxid, Propylenoxid, Cyclohexenoxid und Styroloxid eingesetzt werden.

**7.** Verfahren gemäß Anspruch 1, wobei die Reaktion bei einer Temperatur von ≥ 60 °C bis ≤ 120 °C durchgeführt wird.

**8.** Verfahren gemäß Anspruch 1, wobei der Katalysator vor einem Kontaktieren mit dem Epoxid auf eine Temperatur von ≥ 40 °C bis ≤ 150 °C erwärmt wird.

**9.** Verfahren gemäß Anspruch 1, wobei weiterhin ein Co-Katalysator aus der Gruppe der Ammoniumsalze, Phosphoniumsalze, Bis(triphenylphosphin)iminiumsalze, Amidine, Guanidine oder DMAP in der Reaktion eingesetzt wird.

**10.** Verfahren gemäß Anspruch 1, wobei die Reaktion in Gegenwart eines Alkohols mit ≥1 bis ≤ 8 OH-Gruppen durchgeführt wird.

**11.** Polymerer Carbonatester, erhältlich durch die Reaktion eines cyclischen Epoxids mit Kohlendioxid in einem Verfahren gemäß einem oder mehreren der Ansprüche 1 bis 10, **dadurch gekennzeichnet, dass** das zahlenmittlere Molekulargewicht $M_n$, bestimmt mittels Gelpermeationschromatographie, in einem Bereich von ≥ 500 g/mol bis ≤ 5000 g/mol liegt und der Polydispersitätsindex $M_w/M_n$ in einem Bereich von ≥ 1 bis ≤ 1,5 liegt.

**12.** Carbonatester gemäß Anspruch 12, wobei das cyclische Epoxid Cyclohexenoxid ist.

**13.** Verwendung von Komplexen eines Metalls M oder M-A mit einem Liganden L als Katalysatoren für die Reaktion eines Epoxids mit Kohlendioxid, wobei das Metall M in einer Oxidationsstufe von ≥ 0 vorliegt,
A für Halogenid, Carboxylat, Phenolat, Sulfonat, Phosphonat, Alkyl, Alkoxy oder Amido steht
und der Ligand L die nachfolgende Struktur (Ia) oder (Ib) aufweist, wobei weiterhin eine oder beide der in (Ia) und/oder (Ib) gezeigten OH-Gruppen deprotoniert sein können:

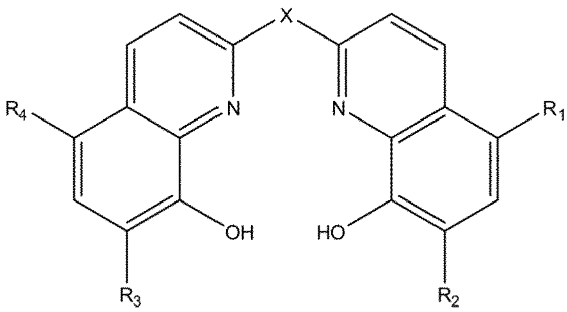

(Ia)

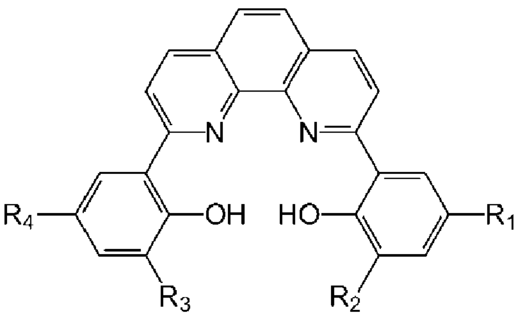

(Ib)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen und X für eine Brücke der Formel (II) oder (III) steht:

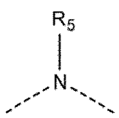

(II)

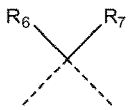

(III)

wobei R5 für einen $C_1$-$C_{22}$-Alkylrest, einen $C_5$-$C_{12}$-Cycloalkylrest, einen $C_7$-$C_{14}$-Aralkyl oder - Alkylarylrest, einen $C_6$-$C_{14}$-Arylrest, oder einen gegebenenfalls Alkyl-substituierten Pyridylrest oder Pyridylmethylrest steht und
R6 und R7 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest oder einen - COOR8-Rest stehen, wobei R8 für einen $C_1$-$C_8$-Alkylrest steht.

**14.** Metallkomplexe gemäß der Formel (XIX), **dadurch gekennzeichnet, dass** das Metall M Cr(III)-A, Co(III)-A und/oder Zn(II) ist und A für Halogenid, Carboxylat, Phenolat, Sulfonat, Phosphonat, Alkyl, Alkoxy oder Amido steht:

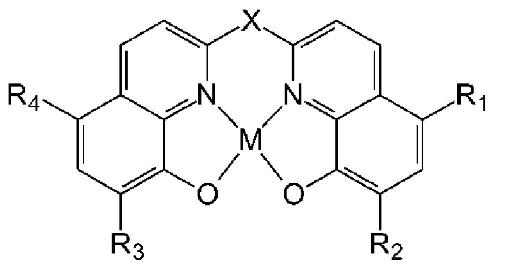

(XIX)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen und X für eine Brücke der Formel (II) oder (III) steht:

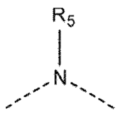

(II)

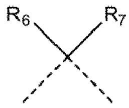

(III)

wobei R5 für einen $C_1$-$C_{22}$-Alkylrest, einen $C_5$-$C_{12}$-Cycloalkykest, einen $C_7$-$C_{14}$-Aralkyl oder - Alkylarylrest, einen $C_6$-$C_{14}$-Arylrest, oder einen gegebenenfalls Alkyl-substituierten Pyridylrest oder Pyridylmethylrest steht und
R6 und R7 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest, einen $C_6$-$C_{14}$-Arylrest oder einen - COOR8-Rest stehen, wobei R8 für einen $C_1$-$C_8$-Alkylrest steht;
oder Metallkomplexe der Formel gemäß der Formel (XX), bei denen das Metall M Cr(III)-A, Co(III)-A und/oder Zn (II) ist und A für Halogenid, Carboxylat, Phenolat, Sulfonat, Phosphonat, Alkyl, Alkoxy oder Amido steht:

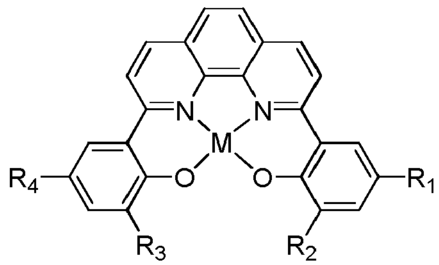

(XX)

wobei R1, R2, R3 und R4 unabhängig voneinander für Wasserstoff, einen $C_1$-$C_{22}$-Alkylrest, einen $C_5$-$C_{12}$-Cycloalkylrest, einen $C_7$-$C_{14}$-Aralkyl oder -Alkylarylrest oder einen $C_6$-$C_{14}$-Arylrest stehen.

**15.** Metallkomplexe gemäß Anspruch 14, ausgewählt aus der Gruppe umfassend Verbindungen der Formeln (XXI) bis (XXVI):

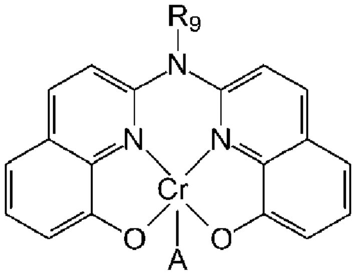

(XXI)

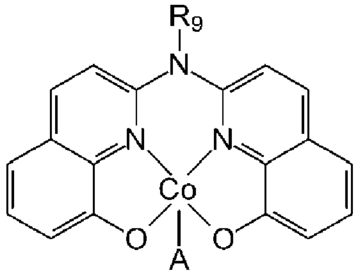

(XXII)

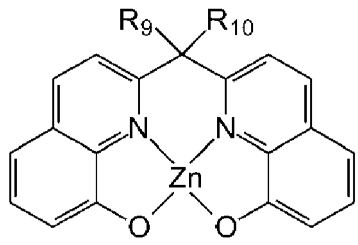

(XXIII)

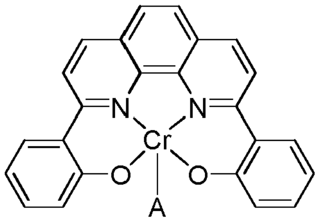

(XXIV)

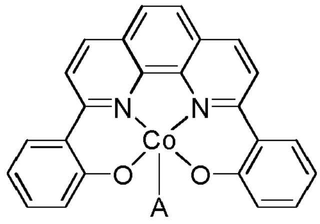

(XXV)

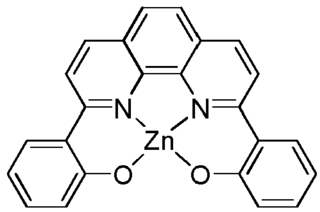

(XXVI)

wobei R9 und R10 unabhängig voneinander für Wasserstoff, Methyl, Ethyl, Propyl, Butyl, Pentyl oder Hexyl stehen und A für $OCOCH_3$, $OCOCF_3$, $OSO_2CF_3$, $OSO_2C_6H_5CH_3$, $OSO_2CF_3$, $OSO(CH_3)_2$ oder Halogenid steht.

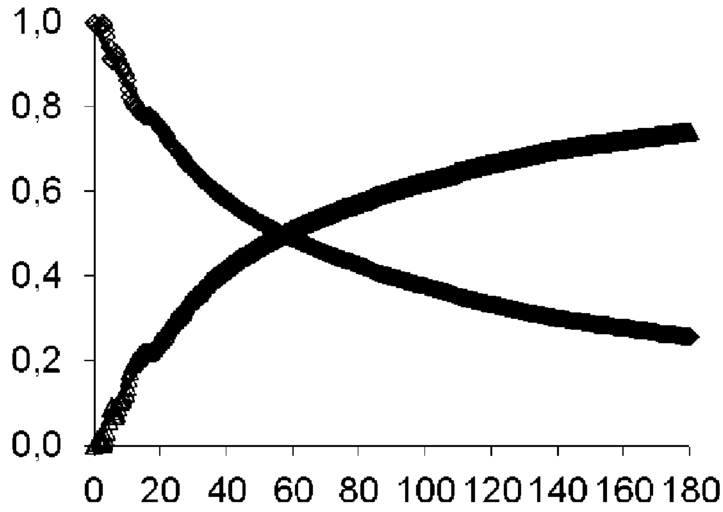
1,0
0,8
0,6
0,4
0,2
0,0
0 20 40 60 80 100 120 140 160 180

FIG. 1

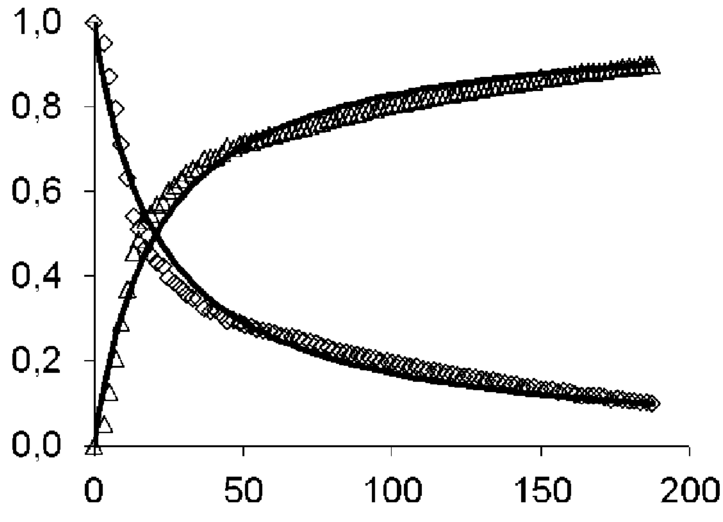
1,0
0,8
0,6
0,4
0,2
0,0
0 50 100 150 200

FIG. 2

Europäisches Patentamt
European Patent Office
Office européen des brevets

# EUROPÄISCHER RECHERCHENBERICHT

Nummer der Anmeldung
EP 12 18 1442

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br><br><br><br><br><br>A | DATABASE WPI<br>Week 200428<br>Thomson Scientific, London, GB;<br>AN 2004-297695<br>XP002690067,<br>& JP 2003 342287 A (MITSUI CHEM INC)<br>3. Dezember 2003 (2003-12-03)<br>* Zusammenfassung *<br>----- | 11,12<br><br><br><br><br><br><br>1-10,<br>13-15 | INV.<br>C07D215/38<br>C07D317/36<br>C07D317/46<br>C07D471/04<br>C08G64/02<br>C08G64/34 |
| X | US 2009/227626 A1 (DERAEVE CELINE [FR] ET AL) 10. September 2009 (2009-09-10)<br>* Komplexe Zn(II)-1 und Zn(II)-3 in Abs. 328 und 330;<br>Ansprüche 23-25 *<br>----- | 14,15 | |
| X | EP 0 964 459 A2 (BAYER AG [DE])<br>15. Dezember 1999 (1999-12-15)<br>* Komplexe B1, B2 und B11 in Abs. 48;<br>Absatz [0042] - Absatz [0047] *<br>----- | 14 | |
| X,D | MICHAEL R. KEMBER ET AL: "Catalysts for CO2/epoxide copolymerisation",<br>CHEMICAL COMMUNICATIONS,<br>Bd. 47, Nr. 1, 1. Januar 2011 (2011-01-01)<br>, Seite 141, XP055007134,<br>ISSN: 1359-7345, DOI: 10.1039/c0cc02207a<br>* das ganze Dokument *<br>----- | 11,12 | RECHERCHIERTE SACHGEBIETE (IPC)<br>C07D<br>C08G |

Der vorliegende Recherchenbericht wurde für alle Patentansprüche erstellt

1

| Recherchenort | Abschlußdatum der Recherche | Prüfer |
|---|---|---|
| Den Haag | 11. Januar 2013 | Mooren, Nicolai |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 12 18 1442

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-01-2013

| Im Recherchenbericht angeführtes Patentdokument | Datum der Veröffentlichung | Mitglied(er) der Patentfamilie | Datum der Veröffentlichung |
|---|---|---|---|
| JP 2003342287 A | 03-12-2003 | KEINE | |
| US 2009227626 A1 | 10-09-2009 | CA 2616453 A1 | 08-02-2007 |
| | | EP 1919894 A2 | 14-05-2008 |
| | | EP 2289891 A2 | 02-03-2011 |
| | | FR 2889525 A1 | 09-02-2007 |
| | | US 2009227626 A1 | 10-09-2009 |
| | | US 2012277265 A1 | 01-11-2012 |
| | | WO 2007015017 A2 | 08-02-2007 |
| EP 0964459 A2 | 15-12-1999 | CA 2273707 A1 | 09-12-1999 |
| | | DE 19825737 A1 | 16-12-1999 |
| | | EP 0964459 A2 | 15-12-1999 |
| | | JP 2000030866 A | 28-01-2000 |
| | | KR 20000005992 A | 25-01-2000 |
| | | TW 432897 B | 01-05-2001 |
| | | US 6294273 B1 | 25-09-2001 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009095164 A1
- WO 2008135337 A1
- DE 102008006881 A1
- DE 20090806 A1
- DE 19981025737 A1


### In der Beschreibung aufgeführte Nicht-Patentliteratur


- *Chemical Communications,* 2011, vol. 47, 141-163
- **OREJON, ARANZAZU ; CASTELLANOS, AIDA ; SALAGRE, PILAR ; CASTILLON, SERGIO ; CLAVER, CARMEN.** *Can. J. Chem.,* vol. 83, 764-768
- **ROUTIER, SYLVAIN ; JOANNY, VALERIE ; ZAPARUCHA, ANNE ; VEZIN, HERVE ; CATTEAU, JEAN-PIERRE ; BERNIER, JEAN-LUC ; BAILLY, CHRISTIAN.** *J. Chem. Soc., Perkin Trans.,* 1998, vol. 2, 863-868